(19) Europäisches Patentamt
European Patent Office
Office européen des brevets

(11) **EP 4 625 255 A1**

(12) **EUROPEAN PATENT APPLICATION**
published in accordance with Art. 153(4) EPC

(43) Date of publication:
**01.10.2025 Bulletin 2025/40**

(21) Application number: **23894480.5**

(22) Date of filing: **14.11.2023**

(51) International Patent Classification (IPC):
**G06N 3/0455** (2023.01)      **G16C 20/70** (2019.01)
**G16C 20/80** (2019.01)

(52) Cooperative Patent Classification (CPC):
**G06N 3/0455; G16C 20/70; G16C 20/80**

(86) International application number:
**PCT/JP2023/040970**

(87) International publication number:
**WO 2024/111471 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 21.11.2022   JP 2022186043
09.08.2023   JP 2023130520

(71) Applicants:
• **OMRON Corporation**
**Kyoto 600-8530 (JP)**
• **The University of Osaka**
**Suita-shi Osaka 565-0871 (JP)**

(72) Inventors:
• **CHIBA, Naoya**
**Tokyo 113-0033 (JP)**

• **USHIKU, Yoshitaka**
**Tokyo 113-0033 (JP)**
• **TANIAI, Tatsunori**
**Tokyo 113-0033 (JP)**
• **IGARASHI, Ryo**
**Tokyo 113-0033 (JP)**
• **ONO, Kanta**
**Suita-shi, Osaka 565-0871 (JP)**
• **SUZUKI, Yuta**
**Suita-shi, Osaka 565-0871 (JP)**
• **SAITO, Kotaro**
**Suita-shi, Osaka 565-0871 (JP)**

(74) Representative: **SONN Patentanwälte GmbH & Co KG**
**Riemergasse 14**
**1010 Wien (AT)**

(54) **INFORMATION PROCESSING DEVICE, TRAINED MODEL GENERATION DEVICE, INFORMATION PROCESSING METHOD, TRAINED MODEL GENERATION METHOD, INFORMATION PROCESSING PROGRAM, AND TRAINED MODEL GENERATION PROGRAM**

(57)    A field that represents the structure of a substance formed with an atomic point cloud is expressed using a neural network model.

FIG.5

Figure 5. Results of interpolation from ZnS to CdS.

**Description**

Technical Field

The present disclosure relates to an information processing device, a trained model generation device, an information processing method, a trained model generation method, an information processing program, and a trained model generation program.

Background Art

There are known techniques relating to autoencoder-based generative deep representation learning pipelines for 3D crystalline structures (for example, see Callum J. Court, Batuhan Yildirim, Apoorv Jain, and Jacqueline M. Cole, "3-D Inorganic Crystal Structure Generation and Property Prediction via Representation Learning", J. Chem. inf. Model. 2020, 60, 10, 4518-4535, [searched November 18, 2022], the Internet <URL: https://pubs.acs.org/doi/10.1021/acs. jcim.0c00464>).

SUMMARY OF INVENTION

Technical Problem

The present disclosure aims to express a field representing the structure of a substance formed with an atomic point cloud.

Solution to Problem

To achieve the above objective, an information processing device according to the present disclosure is an information processing device including a processing unit that expresses a field representing the structure of a substance formed with an atomic point cloud, using a neural network model.

An information processing method according to the present disclosure is an information processing method implemented by a computer to perform a process of expressing a field representing the structure of a substance formed with an atomic point cloud, using a neural network model.

An information processing program according to the present disclosure is an information processing program for causing a computer to perform a process of expressing a field representing the structure of a substance formed with an atomic point cloud, using a neural network model.

Advantageous Effects of Invention

With an information processing device, an information processing method, and an information processing program according to the present disclosure, it is possible to express a field representing the structure of a substance formed with an atomic point cloud.

BRIEF DESCRIPTION OF DRAWINGS

Fig. 1 is a diagram for explaining the present embodiment.

Fig. 2 is a diagram for explaining the present embodiment.

Fig. 3 is a diagram for explaining the present embodiment.

Fig. 4 is a diagram for explaining the present embodiment.

Fig. 5 is a diagram for explaining the present embodiment.

Fig. 6 is a diagram for explaining the present embodiment.

Fig. 7 is a block diagram showing the hardware configuration of an information processing device according to the present embodiment.

Fig. 8 is a diagram for explaining the present embodiment.

Fig. 9 is a table for explaining the present embodiment.

Fig. 10 is a diagram for explaining the present embodiment.

Fig. 11 is a diagram for explaining the present embodiment.

Fig. 12 is a diagram for explaining the present embodiment.

Fig. 13 is a chart for explaining the present embodiment.

Fig. 14 is a chart for explaining the present embodiment.

Fig. 15 is a chart for explaining the present embodiment.

Fig. 16 is a chart for explaining the present embodiment.

Fig. 17 is a chart for explaining the present embodiment.

Fig. 18 is a chart for explaining the present embodiment.

Fig. 19 is a chart for explaining the present embodiment.

Fig. 20 is a chart for explaining the present embodiment.

Fig. 21 is a chart for explaining the present embodiment.

Fig. 22 is a chart for explaining the present embodiment.

Fig. 23 is a chart for explaining the present embodiment.

Fig. 24 is a chart for explaining the present embodiment.

Fig. 25 is a chart for explaining the present embodiment.

Fig. 26 is a chart for explaining the present embodiment.

Fig. 27 is a chart for explaining the present embodiment.

Fig. 28 is a chart for explaining the present embodiment.

Fig. 29 is a chart for explaining the present embodiment.

Fig. 30 is a chart for explaining the present embodiment.

DESCRIPTION OF EMBODIMENTS

In the following, an example of an embodiment of the present disclosure is described, with reference to the drawings. In the present embodiment, an information processing device according to the present disclosure is described as an example. Note that, in the respective drawings, the same or equivalent components and portions are denoted by the same reference numerals. Further, the dimensions and ratios in the drawings are exaggerated for convenience sake, and might be different from actual ratios.

<Overview>

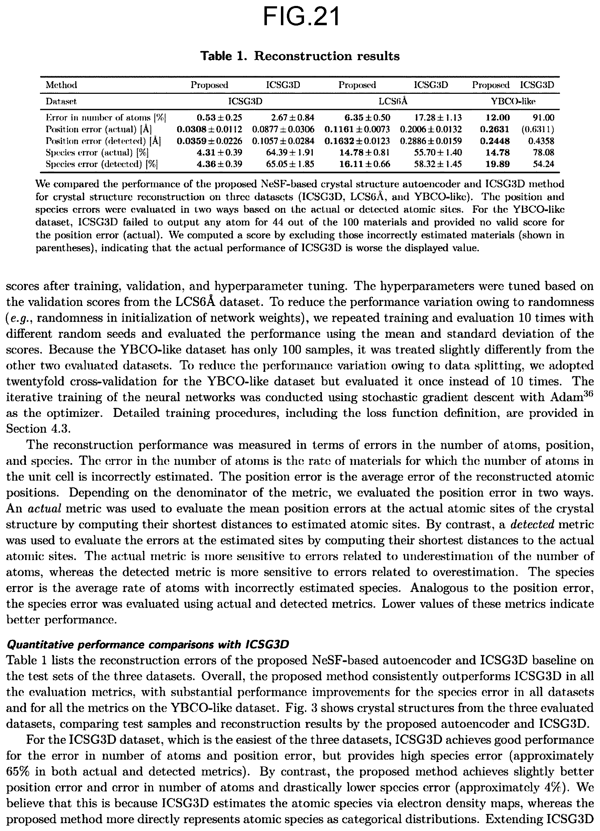

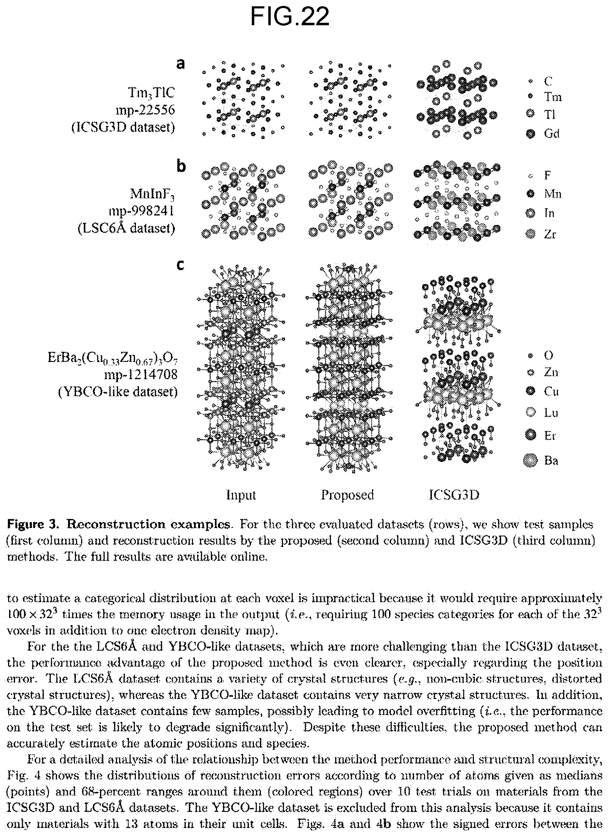

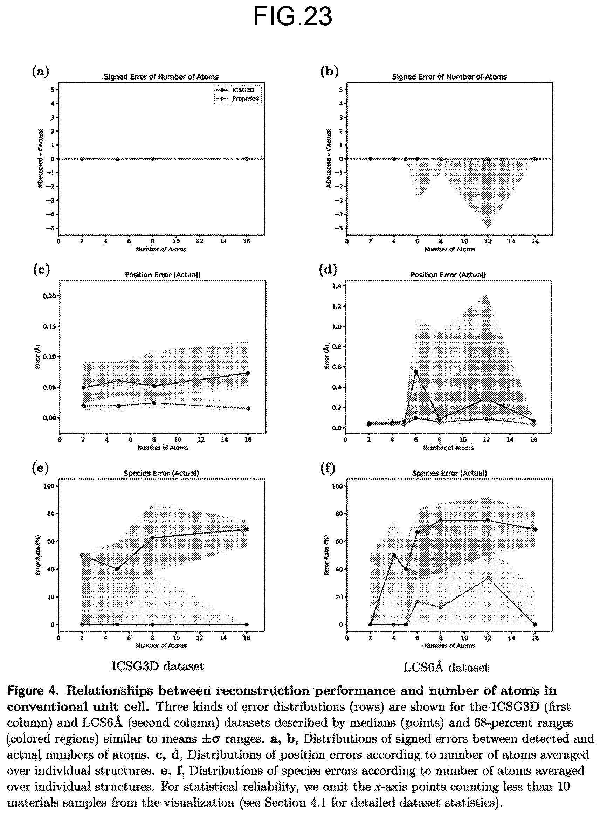

A method of inverse design of crystals, particularly materials, can contribute to next generation methods of searching for materials having the desired properties, without relying on "luck" or "serendipity". In the present embodiment, neural structure fields (NeSF) is proposed as an accurate and practical approach for representing crystal structures using neural networks. Inspired by the concepts of vector fields in physics and implicit neural representations in computer vision, the proposed NeSF considers a crystal structure as a continuous field, rather than a discrete set of atoms. Unlike existing grid-based discretized spatial representations, the NeSF overcomes the tradeoff between spatial resolution and computational complexity, and can represent any crystal structure. To evaluate the NeSF, the present embodiment proposes an autoencoder of crystal structures that can recover various crystal structures, such as those of perovskite structure materials and cuprate superconductors. Extensive quantitative results demonstrate the superior performance of the NeSF compared with the existing grid-based approach. Note that, in the following description, the numbers in brackets "[]" indicate the numbers assigned to the references shown in Figs. 21 to 23.

<1. Introduction>

A fundamental paradigm of materials science considers structure-property relationships assuming that the material properties are tightly coupled with their crystal structures. Therefore, for traditional approaches in materials science, theoretical and experimental analyses of the structure-property relationships of materials are conducted in search for novel materials having superior properties [1, 2]. However, these traditional approaches rely on labor-intensive human analysis and even "serendipity". To automate or assist the material analysis and development, data-driven approaches have been actively studied in materials science and the area of materials informatics (MI) has been established [3-6]. Unlike traditional approaches based on the deduction of physical laws, MI aims to unveil materials knowledge (such as laws governing structure-property relationships) from datasets of collected materials via statistical and machine learning (ML) methods. In recent years, MI has been developed rapidly owing to technological advances in ML and the advent of large-scale materials databases [6, 7]. Thus, powerful neural-network-based ML methods are becoming key components in MI research [6]. Applications of MI include the prediction of material properties from material characteristic data such as crystal structures [6] and compositions [8-11], automated analyses of experimental data [12-14], and natural language processing for knowledge retrieval from scientific literature [15].

Many MI studies have been focused on predicting the properties of given materials, such as the bandgap, Seebeck coefficient, and elastic modulus [16-18]. When this type of task is regarded as the discovery of structure-to-property relationships among materials, there is yet another important type of task, which is the discovery of property-to-structure relationships that constitutes an inverse design problem [19-23].

Despite the potential utility of this inverse approach in developing materials, few studies have addressed this [19-23] or its underlying problem [24-26], that is, the estimation of crystal structures under given conditions. Regarding MI and ML, whether to input or output crystal structures (that is, whether to encode or decode crystal structures in MI and ML terms) induces a crucial difference. Although encoding crystal structures is suitably established using graph neural networks [10, 16, 17, 27], a technical bottleneck remains in decoding crystal structures. In this study, the bottleneck was addressed.

The crystal structure of an inorganic material is a regular and periodic arrangement of atoms in a three-dimensional (3D) space. This arrangement is usually described by the 3D positions and species of atoms in a unit cell and the lattice constants defining the movement of the unit cell in 3D space. The atoms in a unit cell have no explicit order, and their quantity varies from one to hundreds in number. Because ML models, including neural networks, generally accept fixed-dimensional and consistently ordered tensors for processing, handling crystal structures with ML models is not easy [16], and determining crystal structures via the models is even more difficult.

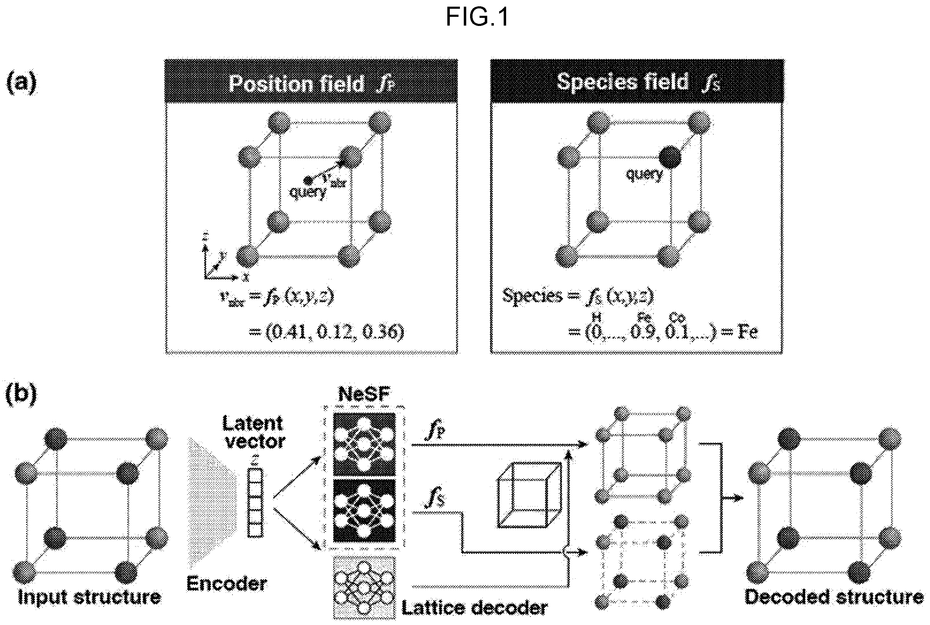

The present embodiment proposes a general representation of crystal structures that enables neural networks to decode or determine such structures. The key concept underlying our approach is illustrated in Fig. 1. Here, a crystal structure is not represented as a discrete set of atoms, but as a continuous vector field tied to 3D space. We refer to our approach as neural structure fields (NeSF). The NeSF uses two types of vector fields, which are the position and species fields, to implicitly represent the positions and species of the atoms in the unit cell of the crystal structure, respectively.

To illustrate the concept of the NeSF, it is assumed that target material information is given as a fixed-dimensional vector z, and the problem of recovering a crystal structure from z is considered. The input z can specify, for example, information about the crystal structure of a material or some desired criteria for materials to be produced. In the NeSF, a neural network f is not made to directly output the crystal structure as f(z), but the neural network f is used as an implicit function to indirectly represent the crystal structure embedded in z. Specifically, f is treated as a vector field on 3D Cartesian coordinates p, conditioned on the target material information z.

[Math. 1]

$$s = f(p, z) \tag{1}$$

In the position field, the network f is trained to output a 3D vector pointing from a query point p to its nearest atomic position a in the crystal structure of interest. Thus, an output s is expected to be a-p. In a case where the position field is ideally trained, the position a of the nearest atom at any query point p can be obtained as p + f(p, z).

Mathematically, the position field can be interpreted as the gradient vector field -φ(p) of the scalar potential shown below.

[Math. 2]

$$\phi(p) = \tfrac{1}{2}\min_i |p - a_i|^2$$

The scalar potential is represented by the squared distance between the query point p and the position $\{a_i\}$ of the nearest atom. Likewise, the species field is trained to output a categorical probability distribution that indicates the species of the nearest atom. Accordingly, the output dimension of the species field is the number of candidate atomic species.

The proposed NeSF is inspired by the concept of vector fields in classical physics and implicit neural representations [30-34] in computer vision. Implicit neural representations have recently been proposed to handle some representation issues in 3D computer vision applications, such as 3D shape estimation of objects [31-33] and free-viewpoint image synthesis [30, 34]. In 3D shape estimation, when a neural network is made to directly output a 3D mesh or point cloud, representation issues similar to those occurring in crystal structures occur. To overcome these issues, the signed distance function (SDF) is utilized in DeepSDF [31] to model 3D shapes by causing the neural network f(p) to indicate whether the query point p is outside or inside the object volume with a positive or negative sign in the respective scalar outputs. The NeSF follows the basic idea of implicit neural representations and further extends it to the estimation of crystal structures described by atomic positions and species. The precise description of atomic positions in crystal structures is of crucial interest in materials science. Thus, the NeSF outputs vectors pointing to the nearest atoms to represent atomic positions more directly than existing implicit neural representations of 3D geometries.

Our idea of representing 3D geometry crystal structures as continuous vector fields has been partially and implicitly explored using grid-based discretization (which is voxelization) in recent MI studies [19, 20, 22, 24-26], but without explicit consideration as discretized vector fields. In those studies, the 3D space in the unit cell is discretized into voxels, and each voxel is then assigned an electron density. The electron density basically represents the presence or absence of an atom around the voxel. However, compared with one-dimensional (such as audio signals) or two-dimensional (such as images) data, the discretization of 3D data suffers considerably from the tradeoff between spatial resolution and computational complexity, in terms of both computational time and memory space. For example, the ICSG3D method [24] uses 32 x 32 x 32 voxels to represent the crystal structures and estimates them using 3D convolutional neural networks (CNNs). Because voxel-based 3D CNNs involve a lot of computation and memory, the resolution of 32 x 32 x 32 voxels is an approximate limit for training a voxel-based model on a standard computing system. Meanwhile, existing crystal structures contain tens or more atoms in their unit cells, or have elongated or distorted unit cells. Therefore, accurately representing diverse crystal structures with voxels requires a sufficiently high resolution. Moreover, voxel-based models can only indirectly provide atomic positions in representations such as peaks in a scalar field of electron densities discretized in the voxel space. The proposed NeSF overcomes the limitations of voxelization. In the NeSF, there is essentially no tradeoff between spatial resolution and required memory. Theoretically, the NeSF can achieve infinitely high spatial resolution with compact (memory- and parameter-efficient) neural networks in place of costly 3D CNNs. In addition, the NeSF can effectively represent any crystal structures including those with elongated or distorted unit cells. Furthermore, the NeSF can directly provide the Cartesian coordinates of the atomic positions rather than peaks of a scalar field. It is believed that the proposed NeSF will break through the technical bottleneck of the MI approach for crystal structure estimation and contribute to the advancement of MI research in this direction.

In the following section, the proposed NeSF is described in detail, and its expressive power for various crystal structures is demonstrated through numerical experiments. Notably, the NeSF successfully recovers various crystal structures, from the relatively basic structures of perovskite materials to the complex structures of cuprate superconductors. Results from extensive quantitative evaluations show that the NeSF outperforms the voxelization approach in ICSG3D [24].

<2 Results and Discussion>

First, the procedures for estimating crystal structures with the NeSF and training the NeSF are described. An autoencoder of crystal structures is then presented as an application of the NeSF. In this autoencoder, crystal structures are embedded into vectors z (called latent vectors) via an encoder, and the NeSF then acts as a decoder to recover the input crystal structures from z. The performance of the NeSF-based autoencoder is quantitatively analyzed by evaluating the reconstruction accuracy, outperforming the voxelization-based ICSG3D baseline. The space of learned vectors z is qualitatively analyzed. With the proposed autoencoder, this analysis shows that the learned space reflects some similarity between crystal structures, instead of merely embedding crystal structures at random.

<2.1 Crystal Structures by NeSF>

When estimation target material information is given as a vector z, estimating the crystal structure from z is estimating the positions and species of the atoms in the unit cell along with the lattice constants. The lattice constants are modeled as lengths a, b, and c and angles $\alpha$, $\beta$, and $\gamma$, and are estimated via simple multilayer perceptrons (MLPs) with the input z. On the other hand, the atomic positions and species are estimated by the position field $f_p$ and the species field $f_s$ of the NeSF, respectively, as described in the previous section. These fields are also implemented as simple MLPs, each taking the query position p and the vector z as inputs and predicting a field value (which is a 3D pointing vector or a categorical probability distribution). An overview of the NeSF network architecture is illustrated in the right part of Fig. 1(b).

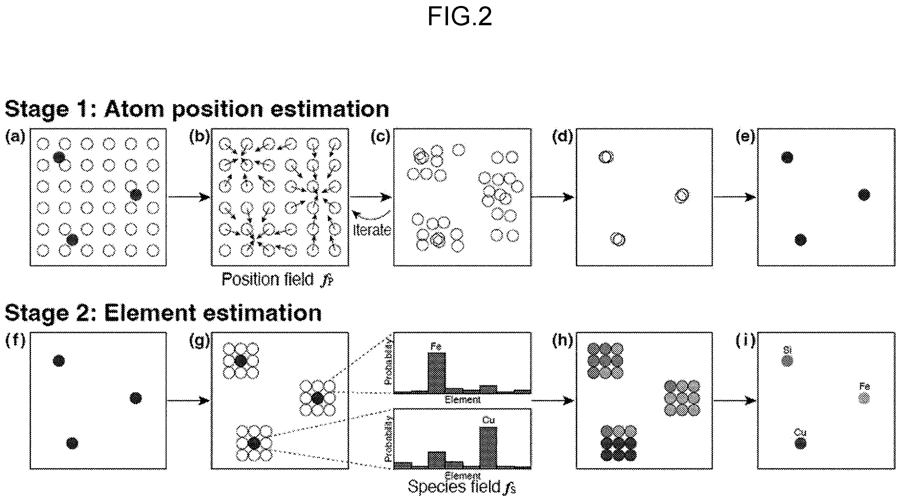

Given the vector z from the encoder, the estimation of the atomic positions and species using the NeSF is illustrated in Fig. 2 and is summarized in five steps.

1. Initialize particles. First, the lattice constants are estimated via MLPs. Initial query points $\{p^0_i\}$ called particles are then regularly spread at 3D grid points within a bounding box. The bounding box is common to each dataset and is given to loosely encompass the atoms of all training samples.

2. Move particles. The position of each particle $p^t_i$ is updated using the position field according to $p^{t+1}_i = p^t_i + f_p(p^t_i, z)$. This process is repeated for all particles, to obtain $\{p_i\}$ as candidate atomic positions. Because the position field is expected to point to the nearest atomic position, the particles travel toward their nearest atoms through this process.

3. Score particles. Each particle $p_i$ is scored, and outliers are filtered. As he norm $\|f_p(p_i, z)\|$ of the output of the position field indicates an estimated distance from $p_i$ to its nearest atom, each particle $p_i$ is scored by $\|f_p(p_i, z)\|$ and, if the score exceeds a certain threshold (set to 0.9 Å in this case), the particle is discarded.

4. Detect atoms. Until this point of time, the particles are expected to form clusters around atoms. Therefore, a simple clustering algorithm is adopted to detect each cluster position as an atomic position, and the number of atoms in the crystal structure is determined. Here, a clustering algorithm well known in object detection and non-maximum suppression is used. Specifically, a list of candidate particles is initialized as $B_c = \{p_i\}$, and another list of accepted particles is initialized as $B_a = \{\}$. 1) The particle with the lowest score (estimated to be nearest to an atom) is selected from $B_c$, and is moved to $B_a$. 2) Particles within a spherical area around the selected particle are removed from $B_c$ (the spherical radius was set to 0.5 Å in this study). These steps are repeated until $B_c$ becomes empty, so that the atomic position $\{ai\}$ as the selected particle stored in $B_a$ is obtained.

5. Estimate species. Finally, the species field $f_s(p, z)$ is used to estimate the atomic species at each atomic position $a_i$. For robust estimation against errors in ai, new particles are intensively spread as queries to the species field around each $a_i$, instead of using $a_i$ directly as a query. Thus, a plurality of probability distributions is obtained, each of the probability distributions predicts the species of an atom $a_i$. The most frequent atomic species among them is selected as the final estimate.

<2.2 Training of NeSF>

The training of the NeSF differs from the above estimation algorithm and is much simpler. Sampling is randomly performed on 3D query points $\{p^s_i\}$ in the unit cell, and loss values for the field outputs at these points are computed. Thus, $f_p(p^s_i, z)$ and $f_s(p^s_i, z)$ are supervised to indicate the position and species of the nearest atom, respectively. However, query points cannot be sampled densely because of practical limitations in memory usage. Therefore, training requires a sampling strategy for query points. Existing implicit neural representations for 3D shape estimation, such as DeepSDF [31], sample the training query points near the surface. Curriculum DeepSDF [35] further introduces curriculum learning, in which the sampling density intensifies near the surface as training proceeds.

To consider a desired sampling strategy for training the position field and the species field, their dynamics are considered in the proposed algorithm. 1) Particles repeatedly move in the position field toward their nearest atoms. Therefore, the position field should be sufficiently accurate anywhere to allow the flow of particles to their destinations, and be highly accurate in the vicinity of atoms. 2) The species field is queried only around atoms. Therefore, the species filed does not need to be accurate everywhere, but should be robust to errors in the estimated atomic positions.

To meet the above requirements, two sampling methods are introduced to train the position field and the species field. 1) Global grid sampling: This method considers 3D grid points that uniformly cover the entire unit cell, and samples the points with perturbations that follow a Gaussian distribution. 2) Local grid sampling: This method considers local 3D grid points centered at each atomic position, and samples the points with perturbations that follow a Gaussian distribution.

To train the position field, the two sampling methods are combined. Accordingly, query points are sampled uniformly over the unit cells and densely around the atoms. To train the species field, local grid sampling is used to concentrate training query points in the vicinity of atoms.

<2.3 Crystal Structure Autoencoder>

To demonstrate and evaluate the expressive power of the NeSF, an autoencoder of crystal structures is proposed. Like other common autoencoders, the proposed NeSF-based autoencoder includes an encoder and a decoder. The encoder is a neural network that transforms an input crystal structure (which is the positions and species of the atoms in the unit cell, and lattice constants) into an abstract latent vector z. The decoder, for which the NeSF is used, reconstructs the input crystal structure from the latent vector z. Autoencoders are typically used to learn latent vector representations of data via self-supervised learning. In this learning, the input data can supervise the learning via a reconstruction loss.

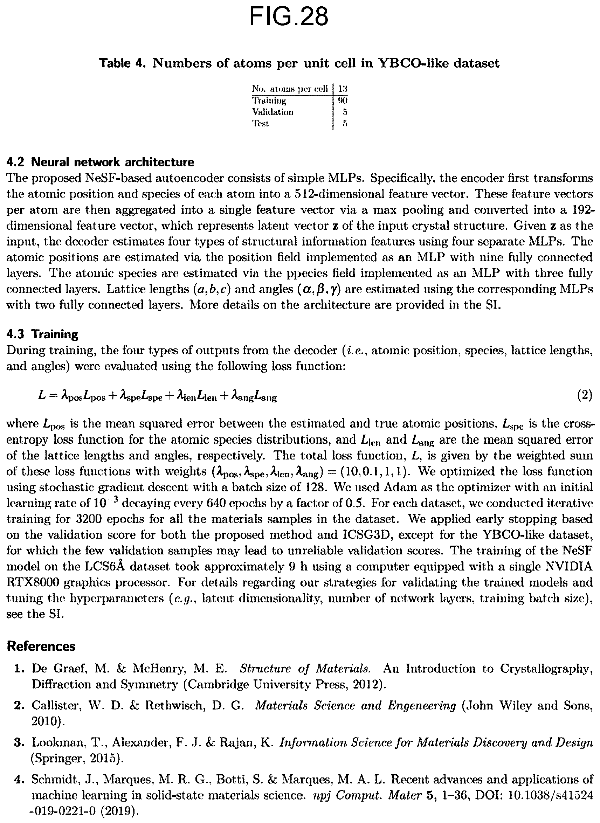

While we focus on decoding crystal structures, their encoding is studied in MI. Because a crystal structure is essentially a set of atoms, its encoding needs to handle a variable number of atoms with invariance to permutation. In ML, such encoders are generally called set functions [28, 29]. Among them, the family of graph neural networks [10, 16, 17, 27] serves as popular crystal structure encoders. However, these networks implicitly represent atomic positions as edges, and encode distances between atoms while discarding the exact coordinates. While this distance-based graph representation is a key to ensuring invariance to coordinate system, its information loss in input might unintentionally hinder the reconstruction performance. Therefore, the basic encoder architecture from PointNet [28] and DeepSets [29] is adopted. This architecture not only represents the simplest type of set-function-based networks but can also preserve information about input crystal structures, and thus, is suitable for evaluation of performance of the NeSF decoders. The architecture of the proposed autoencoder is detailed in Section 4.2 in Fig. 28.

<Training and Evaluation Procedures>

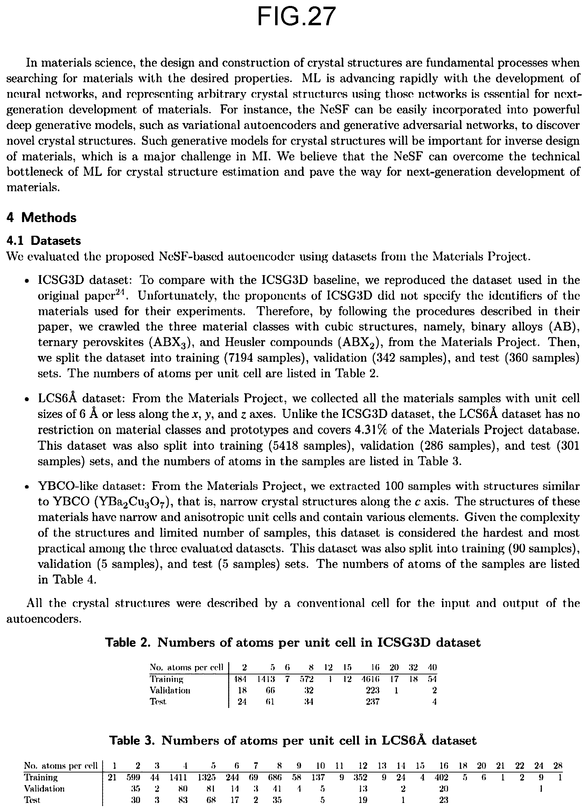

The autoencoder and ICSG3D24 (baseline) were trained and evaluated on three material datasets: ICSG3D, limited cell size 6Å (LCS6Å), and YBCO-like datasets. These datasets are designed to collect the crystal structures of materials from the Materials Project, and have different difficulty levels. The ICSG3D dataset [24] is a materials collection, and includes three datasets containing 7897 materials with limited crystal systems (cubic) and prototypes (which are AB, ABX2, and ABX3). The LCS6Å dataset is formed with 6005 materials having a unit cell size of 6Å or smaller along the x-, y-, and z-axes, and there are no restrictions on the crystal systems and prototypes. The YBCO-like dataset is formed with 100 materials having narrow unit cells along the c-axis. These structures typically include structures of yttrium barium copper oxide (YBCO) superconductors. Due to the complexity of the structure and relatively few samples, the YBCO-like dataset is the most challenging among the three evaluated datasets. Further details on these datasets are provided in Section 4.1 in Fig. 27.

For training and evaluation, each dataset was randomly split into training (90.25%), validation (4.75%), and test (5%) sets. The training set was only used to train the ML models. The validation set was used to preliminarily validate the trained ML model, and the test set was used to compute the final evaluation scores after training, validation, and hyperparameter tuning. The hyperparameters were tuned based on the validation scores from the LCS6Å dataset. To reduce the performance variation due to validation score randomness (such as randomness in initialization of network weights), training and evaluation were repeated 10 times with various random seeds, and the performance was evaluated using the mean and standard deviation of the scores. Because the YBCO-like dataset has only 100 samples, the YBCO-like dataset was processed in a slightly different manner from the other two evaluated datasets. To reduce the performance variation due to data splitting, twentyfold cross-validation was adopted for the YBCO-like dataset, but the YBCO-like dataset was evaluated once, not 10 times. The iterative training of the neural networks was conducted using stochastic gradient descent with Adam [36] as the optimizer. Detailed training procedures, including the loss function definition, are provided in Section 4.3 in Fig. 28.

The reconstruction performance was measured in terms of errors in the number, positions, and species of atoms. The error in the number of atoms is the ratio of materials for which the number of atoms in the unit cell is incorrectly estimated. The position error is the mean error of the reconstructed atomic positions. Depending on the denominator of the metric, the position error was evaluated by two methods. An actual metric was used to evaluate the mean position errors at the actual atomic sites of the crystal structure by computing their shortest distances to estimated atomic sites. In contrast, a detected metric was used to evaluate the errors at the estimated sites by computing their shortest distances to the actual atomic sites. The actual metric is more sensitive to errors related to underestimation of the number of atoms, while the detected metric is more sensitive to errors related to overestimation. The species error is the average rate of atoms whose species are incorrectly estimated. Like the position error, the species error was evaluated using actual and detected metrics. Lower values of these metrics indicate better performance.

<Quantitative Performance Comparisons with ICSG3D>

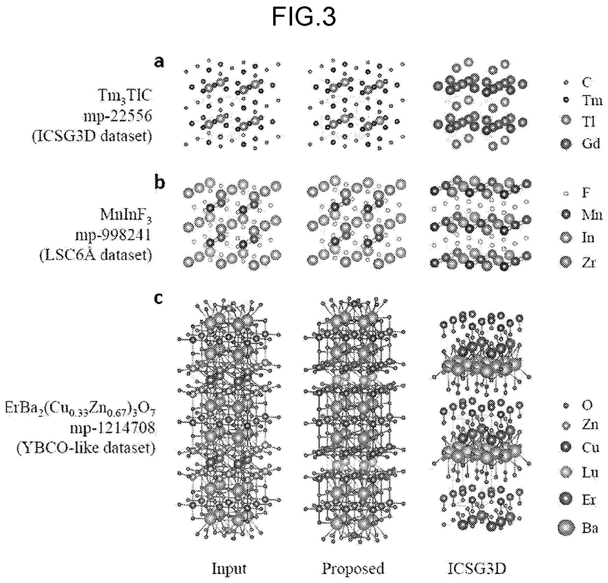

Table 1 shows the reconstruction errors of the proposed NeSF-based autoencoder and ICSG3D baseline on the test sets of the three datasets. Overall, the proposed method consistently outperforms ICSG3D in all the evaluation metrics, with substantial performance improvements for the species error in all datasets and for all the metrics on the YBCO-like dataset. Fig. 3 shows crystal structures from the three evaluated datasets, comparing test samples and reconstruction results by the proposed autoencoder and ICSG3D.

[Table 1]

**Table 1. Reconstruction results**

| Method | Proposed | ICSC3D | Proposed | ICSC3D | Proposed | ICSG3D |
|---|---|---|---|---|---|---|
| Dataset | ICSC3D | | LCS6A | | YBCO-like | |
| Error in number of atoms [%] | **0.53**±0.25 | 2.67±0.84 | **6.35**±0.50 | 17.28±1.13 | **12.00** | 91.00 |
| Position error (actual) [Å] | **0.0308**±0.0112 | 0.0877±0.0306 | **0.1161**±0.0073 | 0.2006±0.0132 | **0.2631** | (0.6311) |
| Position error (detacted [Å] | **0.0359**±0.0226 | 0.1057±0.0284 | **0.1632**±0.0123 | 0.2886±0.0159 | **0.2448** | 0.4358 |
| Species error (acutal) [%] | **4.31**±0.39 | 64.39±1.91 | **14.78**±0.81 | 55.70±1.40 | **14.78** | 78.08 |
| Species error (detected) [%] | **4.36**±0.39 | 65.05±1.85 | **16.11**±0.66 | 58.32±1.45 | **19.89** | 54.24 |

We compared the performance of the proposed NeSF-based crystal structure autuencoder and ICSG3D method for crystal structure reconstruction on three datasets (ICSG3D, LCS6Å, and YBCO-like). The position and species errors were evaluated in two ways based on the actual or detected atomic sites. For the YBCO-like dataset, ICSG3D failed to output any atom for 44 out of the 100 materials and provided no valid score for the position error (actual). We computed a score by excluding those incorrectly estimated materials (shown in parentheses), indicating that the actual performance of ICSG3D is worse the displayed value.

In the case of the ICSG3D dataset, which is the simplest of the three datasets, ICSG3D achieved good performance for the error in number of atoms and the position error, but provides a large species error (about 65% in both actual and detected metrics). In contrast, the proposed method achieves slightly smaller errors in position and in number of atoms, and a dramatically smaller species error (about 4%). This is likely because ICSG3D estimates atomic species via electron density maps, while the proposed method more directly represents atomic species as categorical distributions. Extending ICSG3D to estimate a categorical distribution at each voxel is impractical, because it would require approximately 100 x 323 times the memory usage in the output (that is, 100 species categories are required for every 323 voxels in addition to one electron density map).

In the cases of the LCS6Å and YBCO-like datasets, which are more challenging than the ICSG3D dataset, the performance advantage of the proposed method is even clearer, especially with regard to the position error. The LCS6Å dataset contains a variety of crystal structures (such as non-cubic structures and distorted crystal structures), while the YBCO-like dataset contains very narrow crystal structures. Further, the YBCO-like dataset contains few samples, which might lead to model overfitting (which means that the performance on the test set is likely to deteriorate significantly). Despite these difficulties, the proposed method can accurately estimate the atomic positions and species.

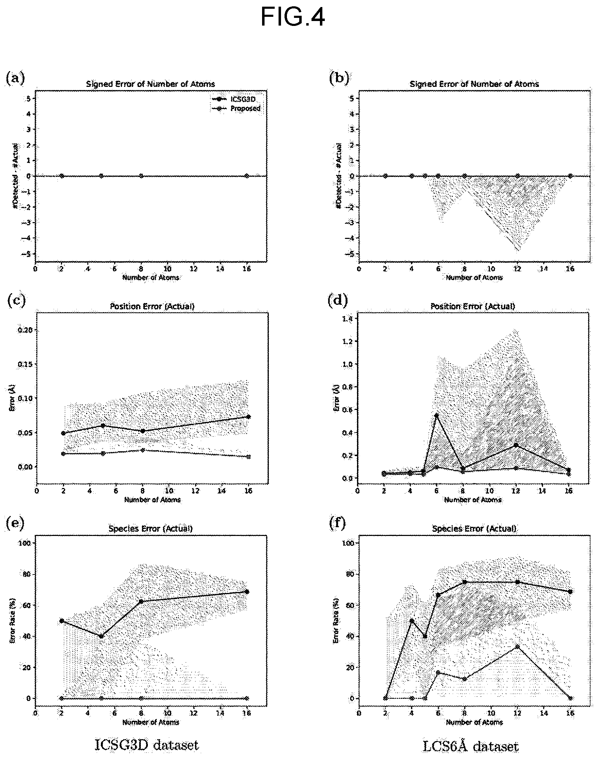

For a detailed analysis of the relationship between method performance and structural complexity, Fig. 4 shows the distributions of reconstruction errors depending on the number of atoms given as medians (points) and the 68% range (colored regions) around them over ten test trials on materials from the ICSG3D and LCS6Å datasets. The YBCO-like dataset is excluded from this analysis, because the YBCO-like dataset contains only materials with 13 atoms in their unit cells. Figs. 4a and 4b show the signed errors between the number of detected atoms and the number of actual atoms. These results indicate that both methods correctly estimate the number of atoms for most (68% or more) of the samples in the ICSG3D dataset (Fig 4a). However, the ICSG3D method underestimates the number of atoms for the LCS6Å dataset (Fig. 4b). Likewise, Figs. 4c and 4d show the distributions of position errors, and Figs. 4c and 4d show the distributions of position error. Figs. 4e and 4f show the distributions of species errors. Because the number of atoms is either correctly estimated or underestimated by both methods in most cases, errors are reported in the actual metrics. By examining the distributions at x = 2 in Fig. 4e, it is possible to determine the tendency of species errors for the diatomic structures in the ICSG3D dataset.

While the proposed method provides the correct species of both atoms for 68% or more of the diatomic materials, ICSG3D often misestimates the species of one of the two atoms. Overall, the three types of errors by both methods tend to increase with the number of atoms, but the proposed method consistently achieves better results than ICSG3D for materials with varying numbers of atoms. Compared with our method, the performance of ICSG3D tends to deteriorate more notably for materials with large numbers of atoms. ICSG3D tends to underestimate the number of atoms (Fig. 4b), which is likely due to the spatial resolution of ICSG3D limited to 32 x 32 x 32 voxels. This suggests that ICSG3D is incapable of capturing polyatomic structures.

It is believed that the notably high performance of the proposed method is attributable to two reasons. First, because our method does not use discretization, it is advantageous over the grid-based ICSG3D for estimating complex crystal structures. In a grid-based method, the spatial resolution is limited by the cubically increasing computations and memory usage. The proposed NeSF is free from such tradeoff between resolution and computational complexity, and thus, is able to effectively represent complex structures. Second, the model size of the proposed NeSF using MLPs is much smaller than the model size of the 3D CNN architecture of ICSG3D. In general, the number of training samples required for an ML model correlates with the number of trainable parameters. Grid-based methods use layers of 3D convolution filters that involve many trainable parameters. In contrast, the NeSF uses implicit neural representations to indirectly describe the 3D space as a field rather than voxels. Thus, the NeSF is efficiently implemented by MLPs with fewer parameters than a 3D CNN. Specifically, the NeSF-based autoencoder has 760,000 parameters, which is only 2.24% of the number of parameters in the 3D CNN-based ICSG3D (34 million parameters). This difference makes the NeSF advantageous over grid-based methods, especially on small datasets such as the YBCO-like dataset.

<2.4 Latent Space Interpolation>

The characteristics of the proposed NeSF-based autoencoder were qualitatively analyzed by inspecting the learned latent space of the crystal structures. In general, a good latent space should map similar items (in terms of properties, characteristics, categories, and the like) closely in the space. In this manner, latent data representations that facilitate human and machine analyses are provided. To assess the construction of the latent space of crystal structures, transitions in the latent space were visualized as sequences of crystal structures. In a case where the latent space is trained to capture relationships between materials in terms of structural similarity, interpolating between two points in the latent space should produce a sequence of materials with similar crystal structures.

Interpolation analysis proceeds as follows.

1. Set the ICSG3D test set as the source and destination materials, and obtain their latent vectors as $z_{src}$ and $z_{dst}$ via the trained encoder.
2. Interpolate between $z_{src}$ and $z_{dst}$ linearly in the latent space to obtain a sequence of latent vectors.
3. Decode each latent vector via the trained decoder (NeSF) to obtain its crystal structure.

Because the intermediate crystal structures between the source and the destination is reconstructed from the latent vector via the trained decoder, these structures might not appear in the dataset.

To facilitate interpretation of the analysis, the well-known zinc-blende and rock-salt structure families were selected as benchmark materials. Both families have compositions given by AX, where A is a cation and X is an anion, and the crystal structure is based on a cubic crystal system. Therefore, in a case where the source and destination materials belong to one of these families, the characteristic composition and structural prototype need to be preserved throughout the interpolation path.

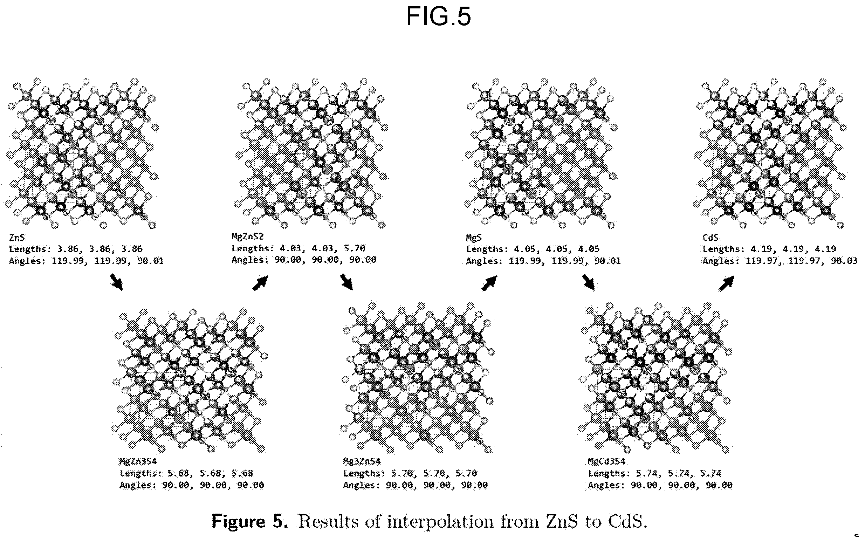

As a first example, Fig. 5 shows the results of interpolation from ZnS (mp-10695) to CdS (mp-2469). The obtained transition in the compositional formula is $ZnS \rightarrow MgZn_3S_4$ $(Mg_{0.25}Zn_{0.75}S) \rightarrow MgZnS_2$ $(Mg_{0.5}Zn_{0.5}S) \rightarrow Mg_3ZnS_4$ $(Mg_{0.75}Zn_{0.25}S) \rightarrow MgS \rightarrow MgCd_3S_4$ $(Mg_{0.25}Cd_{0.75}S) \rightarrow CdS$.

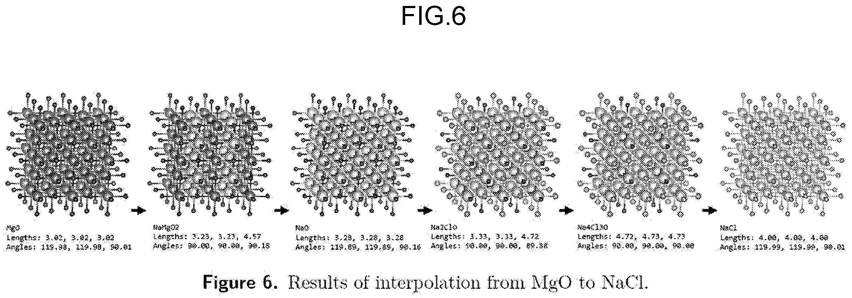

As a second example, Fig. 6 shows the results of interpolation from MgO (mp-1265) to NaCl (mp-22862). The obtained transition in the compositional formula is $MgO \rightarrow NaMgO_2$ $(Na_{0.5}Mg_{0.5}O) \rightarrow NaO \rightarrow Na_2ClO$ $(NaCl_{0.5}O_{0.5}) \rightarrow Na_4C_{13}O$ $(NaCl_{0.75}O_{0.25}) \rightarrow NaCl$.

Further, in the SI, the results of interpolation from NaCl (mp-22862) to PbS, from MgO (mp-1265) to CaO (mp-2605), and from PbS (mp-21276) to CaO (mp-2605) are provided.

In the examples of interpolation, the composition AX and the cubic structure are mostly preserved. Furthermore, the compositions change continuously without collapsing along the interpolation paths. These results suggest that our encoder learns meaningful continuous representations of crystal structures by capturing their characteristics in an abstract space, and the proposed NeSF model successfully decodes these representations into crystal structures.

<2.5 Limitations and Future Directions>

This study was mainly focused on developing a fundamental approach for crystal structure estimation using implicit neural representations. To suggest room for further improvement and important directions in future work, three main limitations of the NeSF were identified.

First, the NeSF adopts a relatively simple network architecture, and architectural design choices were not thoroughly explored. For example, the NeSF treats the elements as mutually independent categorical (one-hot) vectors. Thus, there is no explicit training of the model using the physical characteristics of elements or their similarities. Meanwhile, other developments have attempted to explicitly inject physical characteristics and functions of elements into their models. For example, existing crystal-structure encoders [16] represent input elements using their fingerprints, such as their groups and periods, electron densities, atomic radii, and electronegativities, instead of using one-hot vectors. In ICSG3D24, the outputs of atomic species are trained using the mean square error of the atomic numbers rather than a categorical loss, which is considered in our method. Incorporating these schemes into the model might be able to reflect the characteristics and similarities between elements in the latent space and reconstructed crystal structures. Another architectural choice regarding the NeSF is whether to use the existing (current choice) or primitive cell to represent crystal structures. Both formats have their advantages and drawbacks depending on the application. While the existing unit cell provides more intuitive visualizations that facilitate manual analysis, the primitive unit cell provides more compact structure representations that might facilitate computer processing. In future work, the network architecture design needs to be thoroughly analyzed.

Another limitation is that the proposed NeSF does not explicitly consider space-group symmetry. Therefore, the local spatial arrangements of atoms in the existing unit cells estimated by the NeSF do not necessarily obey the space-group symmetry. Although ICSG3D [24] shares the same limitation, symmetry is an important concept in crystallography. Therefore, incorporating the constraint of space-group symmetry into the NeSF is an important direction of future work.

Finally, for an evaluation purpose, the autoencoder architecture was adopted, instead of generative models such as variational autoencoders [16, 25, 37] and generative adversarial networks [26, 37-39]. These generative models intentionally perturb latent structural representations to produce diverse structures that do not appear in the dataset. While this aspect of the generative models is more suitable for novel structure discovery, the lack of ground-truth structures prevents quantitative and reliable performance analysis.

The NeSF needs to be applied to generative models in future work using appropriate performance analysis.

<3 Conclusion>

In the present embodiment, the NeSF to estimate crystal structures using neural networks is proposed. It is difficult to directly determine crystal structures using neural networks. This is because these structures are essentially represented as an unordered set including varying numbers of atoms. The NeSF overcomes this difficulty by treating the crystal structure not as a discrete set of atoms, but as a continuous vector field. The idea of the NeSF is borrowed from vector fields in physics and the recent implicit neural representations in computer vision. An implicit neural representation is an ML technique to represent 3D geometries using neural networks. The NeSF extends this technique by introducing the position field and the species field to estimate the atomic positions and the species of crystal structures, respectively. Unlike existing grid-based approaches for representing crystal structures, the NeSF is free from the tradeoff between spatial resolution and computational complexity, and can represent any crystal structure.

The NeSF was applied as an autoencoder for crystal structures, and demonstrated its performance and expressive power on datasets having diverse crystal structures. A quantitative performance analysis showed a clear advantage of the NeSF-based autoencoder over an existing grid-based method, especially for estimating complex crystal structures. Furthermore, a qualitative analysis of the learned latent space revealed that the autoencoder does not randomly map crystal structures, but rather captures similarities between crystal structures.

In materials science, the design and construction of crystal structures are fundamental processes in searching for materials having the desired properties. ML is advancing rapidly with the development of neural networks, and representing any crystal structures using those networks is essential for next-generation development of materials. For example, the NeSF can be easily incorporated into powerful deep generative models such as variational autoencoders and generative adversarial networks, and discover new crystal structures. Such generative models of crystal structures will be important for inverse design of materials, which is a major challenge in MI. The NeSF can overcome the technical bottleneck of ML in crystal structure estimation, and pave the way to next-generation development of materials.

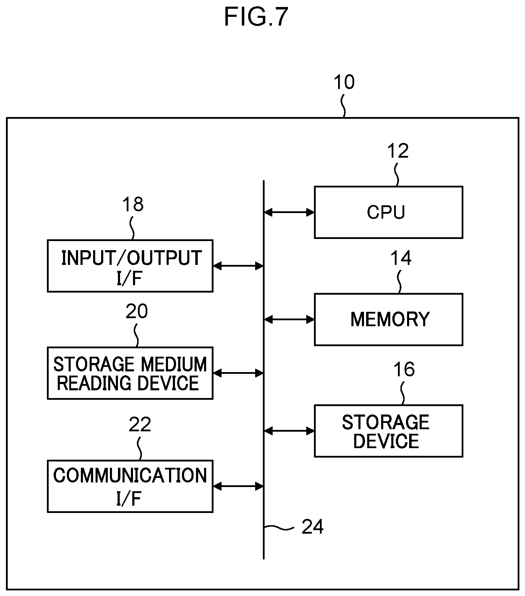

Fig. 7 is a block diagram showing the hardware configuration of an information processing device 10 according to the present embodiment. As illustrated in Fig. 7, the information processing device 10 includes a central processing unit (CPU) 12, a memory 14, a storage device 16, an input/output interface (I/F) 18, a storage medium reading device 20, and a communication I/F 22. The respective components are communicably connected to one another via a bus 24.

The storage device 16 stores an information processing program for executing each of the processes described later. The CPU 12 is a central computing unit, and executes various programs, and controls the respective components. That is, the CPU 12 reads a program from the storage device 16, and executes the program, using the memory 14 as a work area. The CPU 12 performs the above-described various types of arithmetic processing in accordance with the programs stored in the storage device 16.

The memory 14 is formed with a random access memory (RAM), and, as a work area, temporarily stores a program and data. The storage device 16 is formed with a read only memory (ROM), and a hard disk drive (HDD), a solid state drive (SSD), or the like, and stores various programs including an operating system and various kinds of data.

The input/output I/F 18 is an interface that inputs data from an external device, and outputs data to an external device. Further, an input device for performing various inputs, such as a keyboard or a mouse, and an output device for outputting various kinds of information, such as a display or a printer, may be connected to the input/output I/F 18. A touch panel display may be adopted as an output device, and the input/output I/F 18 may be made to function as an input device.

The storage medium reading device 20 reads data stored in various kinds of storage media such as a compact disc (CD)-ROM, a digital versatile disc (DVD)-ROM, a Blu-ray disc, and a universal serial bus (USB) memory, and writes data into a storage medium, for example.

The communication I/F 22 is an interface for communicating with other devices, and a standard such as Ethernet (registered trademark), FDDI, or Wi-Fi (registered trademark) is used.

The information processing device 10 according to the present embodiment trains an autoencoder including an encoder and a decoder, using a technique as described above. As a result, it is possible to express a field representing the structure of a substance formed with an atomic point cloud, using a neural network model.

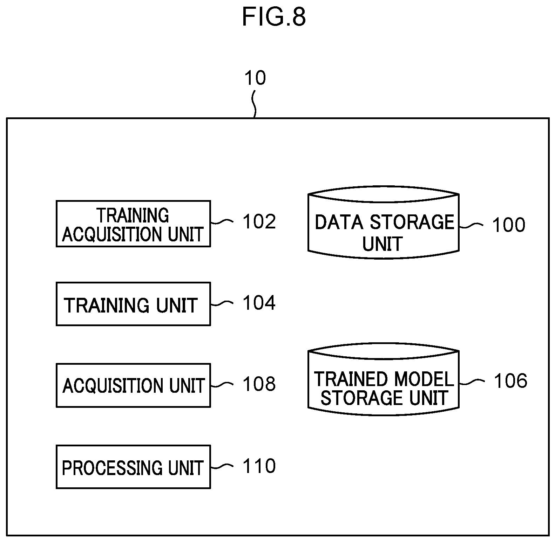

Next, functional components of the information processing device 10 are described. As illustrated in Fig. 8, the information processing device 10 functionally includes a training acquisition unit 102, a training unit 104, an acquisition unit 108, and a processing unit 110. Furthermore, a data storage unit 100 and a trained model storage unit 106 are provided in a predetermined storage area in the information processing device 10. The respective functional components are implemented by the CPU 12 reading the respective programs stored in the storage device 16, loading the programs into the memory 14, and executing the programs.

First, the information processing device 10 trains a neural network model expressing a field representing the structure of a substance formed with an atomic point cloud.

The data storage unit 100 stores training crystal data representing crystal structures of substances. The training crystal data includes position data indicating positions of atoms constituting crystals of substances, species data representing species of atoms constituting the crystals of the substances, and lattice constant data of the crystals of the substances.

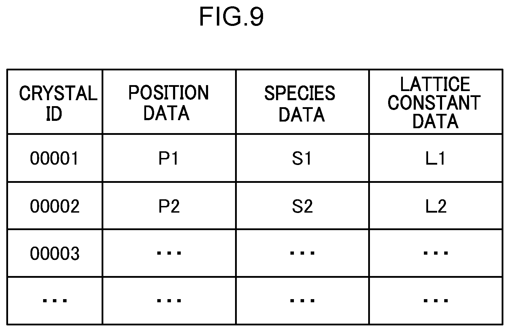

Fig. 9 is a table showing an example of a plurality of sets of training crystal data stored in the data storage unit 100. As shown in Fig. 9, one set of training crystal data is data representing a crystal structure of a substance. As shown in Fig. 9, in one set of training crystal data, position data indicating the positions of atoms constituting the crystal of a substance, species data representing the species of atoms constituting the crystal of the substance, and lattice constant data of the crystal of the substance are stored and are associated with one another. The position data is three-dimensional position coordinate data of the respective atoms in the plurality of atoms. The species data is label data indicating the species of the respective atoms in the plurality of atoms. The lattice constant data includes the length of the crystal axis and the inter-axial angle.

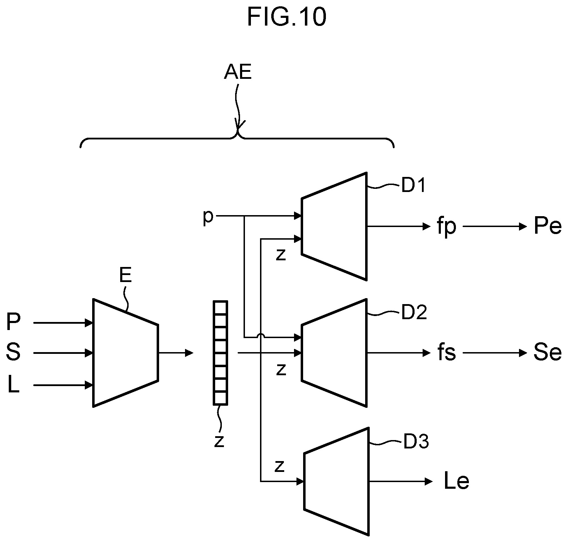

Fig. 10 is a diagram for explaining the structure of an autoencoder of the present embodiment, and an outline of a process to be performed by the information processing device 10 of the embodiment. Fig. 10 is a diagram in which Fig. 1(b) is further simplified.

As shown in Fig. 10, an autoencoder AE of the present embodiment includes an encoder E, a first decoder D1, a second decoder D2, and a third decoder D3.

As shown in Fig. 10, when a combination of position data P indicating the positions of the atoms constituting the crystal of a substance, species data S indicating the species of the atoms constituting the crystal of the substance, and lattice constant data L of the crystal of the substance is input, the encoder E outputs a latent vector z. One set of crystal data indicating the combination of the position data P, the species data S, and the lattice constant data L is set for the crystal structure of one substance.

Also, as shown in Fig. 10, when a combination of a query point p as the attention point in the substance and the latent vector z is input, the first decoder D1 outputs a position field $f_p$ indicating the field of the positions of the atoms constituting the crystal of the substance. On the basis of this position field $f_p$, estimated position data $P_e$ of the atoms constituting the crystal of the substance is computed. The computation method will be described later.

Further, as shown in Fig. 10, when a combination of the query point p as the attention point in the substance and the latent vector z is input, the second decoder D2 outputs a species field $f_s$ indicating the field of the species of the atoms constituting the crystal of the substance. On the basis of this species field $f_s$, estimated species data $S_e$ of the atoms constituting the crystal of the substance is computed. The computation method will be described later.

Also, as shown in Fig. 10, when the query point p as the attention point in the substance and the latent vector z are input, the third decoder D3 outputs estimated lattice constant data $L_e$. Although a case where a single third decoder D3 is adopted is described as an example in the present embodiment, there may be two third decoders D3. In this case, the third decoders D3 are a decoder that outputs the length of the crystal axis, and a decoder that outputs the inter-axial angle, for example.

The information processing device 10 of the present embodiment trains each parameter of the autoencoder AE by unsupervised machine learning (alternatively, self-supervised learning) so that the combination of the position data P, the species data S, and the lattice constant data L input to the autoencoder AE matches the combination of the estimated position data $P_e$, the estimated species data $S_e$, and the estimated lattice constant data $L_e$ output from the autoencoder AE. As a result, a trained autoencoder AE is obtained. Also, a trained first decoder D1, a trained second decoder D2, and a trained third decoder D3, which are components of the trained autoencoder AE, are obtained.

Receiving an instruction signal to train the autoencoder AE, the training acquisition unit 102 reads the training crystal data stored in the data storage unit 100. The training acquisition unit 102 also sets a training query point that is an attention point in the learning substance. Note that the training acquisition unit 102 may set the training query point by randomly sampling positions in the space in a substance for training.

When training the autoencoder AE using unsupervised machine learning, the training unit 104 obtains the latent vector z representing the crystal structure of the substance for training, by inputting the training crystal data to the encoder E in the autoencoder AE. Note that the encoder E can be formed by using the idea of the above-described PointNet [28] or DeepSets [29], for example.

Successively, the training unit 104 inputs the combination of the latent vector z and the training query point p to the first decoder D1 of the autoencoder AE, and thus, obtains the position field $f_p$ representing the field of the positions of the atoms constituting the crystal of the substance for training. The training unit 104 then estimates the positions of the atoms constituting the crystal of the substance for training, on the basis of the position field $f_p$ output from the first decoder D1.

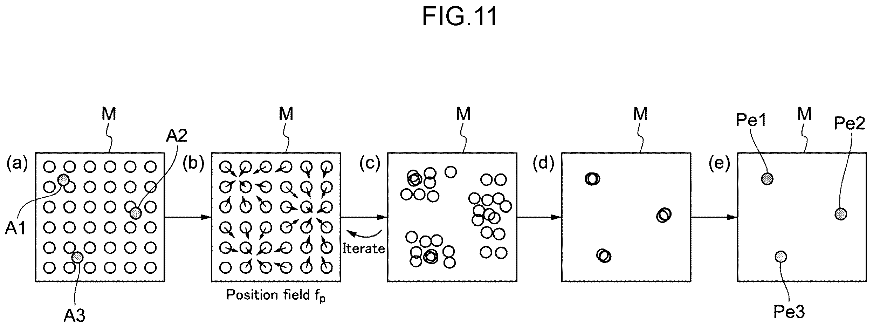

Fig. 11 is a diagram for explaining the setting of the training query point and the position field. Note that Fig. 11 is a diagram similar to one in Fig. 2, but is referred to again herein for ease of explanation. As illustrated in Fig. 11, a plurality of training query points is set in a space M in the substance. White circles shown in Fig. 11 represent the training query points. A1, A2, and A3 shown in Fig. 11(a) represent actual positions of atoms in the space M in the substance.

The training unit 104 obtains the position field $f_p$ as shown in Fig. 11(b) by inputting the combination of the training query point p and the latent vector z to the first decoder D1. Each of the arrows shown in Fig. 11 corresponds to the position field $f_p$.

As illustrated in Figs. 11(b) and 11(c), the training unit 104 then iterates updating of the position of each query point among the plurality of training query points, in accordance with the position field $f_p$ output from the first decoder D1. Specifically, the training unit 104 generates a new position of the training query point by adding a vector represented by the position field $f_p$ to the position of each query point among the plurality of training query points. As a result, the positions of the respective query points among the plurality of training query points converge to the positions of actual atoms, as illustrated in Fig. 11(d). On the basis of the respective positions of the training query points, the training unit 104 obtains estimated positions $P_e1$, $P_e2$, and $P_e3$ of atoms as shown in Fig. 11(e), using Non-max Suppression, for example.

Successively, the training unit 104 inputs the combination of the latent vector z and the training query point p to the second decoder D2 of the autoencoder AE, and thus, obtains the species field fs representing the field of the species of the atoms constituting the crystal of the substance for training. The training unit 104 then estimates the species of the atoms constituting the crystal of the substance for training, on the basis of the species field fs output from the second decoder D2.

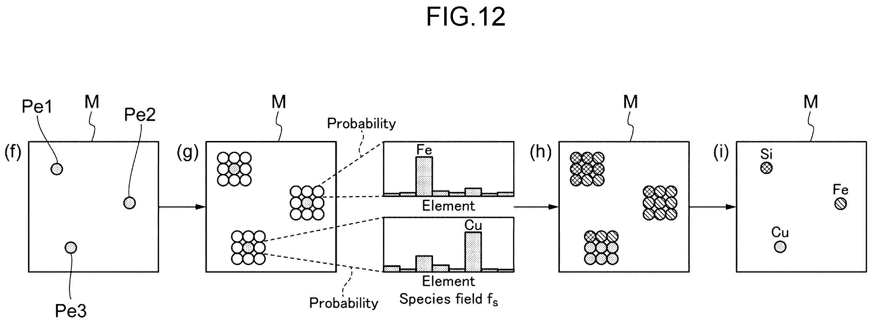

Fig. 12 is a diagram for explaining the setting of the training query point and the species field. Note that Fig. 12 is a diagram similar to one in Fig. 2, but is referred to again herein for ease of explanation. As illustrated in Fig. 12, a plurality of training query points is set in the space M in the substance. White circles shown in Fig. 12 represent the training query points, as in Fig. 12.

Specifically, as illustrated in Figs. 12(f) and 12(g), the training unit 104 sets a plurality of training query points for the positions of estimated positions $P_e1$, $P_e2$, and $P_e3$ of atoms, and positions around the estimated positions $P_e1$, $P_e2$, and $P_e3$.

The training unit 104 then obtains the species field $f_s$ by inputting the combination of the training query point p and the latent vector z to the second decoder D2. As illustrated in Fig. 12(g), the species field $f_s$ corresponds to the probability of indicating the atomic species obtained at each training query point. The example illustrated in Figs. 12(g) and 12(h) show that the probability that the species of the atom located at a training query point is iron Fe is the highest, and the probability that the species of the atom located at another training query point is copper Cu is the highest.

As illustrated in Figs. 12(g) and 12(h), the training unit 104 then estimates the species of the atoms corresponding to the respective positions of the training query points, in accordance with the species field $f_s$ output from the second decoder D2. For example, for each of the estimated positions $P_e1$, $P_e2$, and $P_e3$, the training unit 104 identifies the element having the highest probability at the training query point corresponding to the estimated position, and the element having the highest probability at the training query points around the estimated position. The training unit 104 then estimates the most frequently appearing element at the plurality of training query points set for each of the estimated positions $P_e1$, $P_e2$, and $P_e3$ as the species of the atoms at the estimated positions $P_e1$, $P_e2$, and $P_e3$. As a result, an estimated species $S_e1$ of the atom present at the estimated position $P_e1$, an estimated species $S_e2$ of the atom present at the estimated position $P_e2$, and an estimated species $S_e3$ of the atom present at the estimated position $P_e3$ are obtained.

For example, as illustrated in Fig. 12(i), the species of the atom located at the estimated position $P_e1$ is estimated to be silicon Si, the species of the atom located at the estimated position $P_e2$ is estimated to be iron Fi, and the species of the atom located at the estimated position $P_e3$ is estimated to be copper Cu.

Successively, the training unit 104 obtains lattice constant data $L_e$ of the crystal of the substance for training, by inputting the latent vector z to the third decoder D3 of the autoencoder AE. The lattice constant data $L_e$ output from the third decoder D3 includes the length of the crystal axis and the inter-axial angle.

The training unit 104 then trains the autoencoder AE using unsupervised machine learning so that the combination of the estimated atomic positions, the estimated atomic species, and the lattice constant data output from the third decoder D3 corresponds to the combination of the atomic positions, the atomic species, and the lattice constant data in the training crystal data, and thus, produces the trained first decoder D1 and the trained second decoder D2.

For example, in the above example, the training unit 104 trains the autoencoder AE using unsupervised machine learning so that the estimated positions $P_e1$, $P_e2$, and $P_e3$ of atoms match the position data P1, P2, and P3 of atoms in the training crystal data, the estimated species $S_e1$, $S_e2$, and $S_e3$ of atoms match the species data S1, S2, and S3 of atoms in the training crystal data, and the lattice constant data $L_e$ output from the third decoder D3 matches the lattice constant data L in the training crystal data, and thus, produces the trained first decoder D1 and the trained second decoder D2.

The training unit 104 stores the trained autoencoder AE into the trained model storage unit 106. Because the trained autoencoder AE also includes the trained first decoder D1, the trained second decoder D2, and the trained third decoder D3, these trained models are also stored into the trained model storage unit 106.

Note that the trained encoder E outputs the latent vector z representing the crystal of the substance when crystal data is input, the crystal data being crystal data representing the crystal structure of the substance and including position data indicating the positions of the atoms constituting the crystal of the substance, species data indicating the species of the atoms constituting the crystal of the substance, and lattice constant data of the crystal of the substance.

As described above, the field data representing the field of the structure of the substance of the present embodiment is indicated by the position field representing the field of the positions of the atoms constituting the crystal of the substance and the species field representing the field of the species of the atoms constituting the crystal of the substance. Therefore, when an arbitrary vector and a query point are input, the trained first decoder D1, which is an example of the trained first neural network model, outputs the position field $f_p$ corresponding to the query point. Also, when an arbitrary vector and a query point are input, the second decoder D2, which is an example of the trained second neural network model, outputs the species field $f_s$ corresponding to the query point. Note that the trained first decoder D1 and the trained second decoder D2 are an example of the trained neural network model that outputs field data representing the field of the structure of the substance at a query point when the arbitrary vector and the query point are input.

When the combination of an arbitrary vector in place of the latent vector z and a query point is input, the trained third decoder outputs the lattice constant data $L_e$ of the crystal of the substance.

Receiving an instruction signal to obtain the field data of the crystal structure of the target substance, the acquisition unit 108 reads the trained first decoder D1 and the trained second decoder D2 stored in the trained model storage unit 106. The acquisition unit 108 also acquires the target vector input from the user and a query point which is an attention point in the substance.

Note that the target vector is an arbitrary vector in place of the latent vector z, and may be any vector that describes the properties of the material in the same manner as a latent vector does. For example, the target vector may be a vector representing physical properties desired by the user. For example, [bandgap = xxx] may be stored in a first component of the target vector, and [formation energy = xxx] may be stored in a second component. In this case, the trained first decoder D1 and the trained second decoder D2 are treated as preliminary trained models, and are retrained by a vector representing the physical properties of substances. Thus, useful field data is output from the trained first decoder D1 and the trained second decoder D2.

The processing unit 110 obtains the field data corresponding to the query point by inputting the target vector and the query point acquired by the acquisition unit 108 to the trained first decoder D1 and the trained second decoder D2.

Specifically, the processing unit 110 obtains the position field $f_p$ corresponding to the query point by inputting the target vector and the query point acquired by the acquisition unit 108 to the trained first decoder D1. The processing unit 110 also obtains the species field $f_s$ corresponding to the query point by inputting the target vector and the query point acquired by the acquisition unit 108 to the trained second decoder D2.

The field data output from the trained first decoder D1 and the trained second decoder D2 is data that represents the field of the structure of the substance in accordance with the target vector and the query point. By inputting the target vector that is the arbitrary vector in place of the latent vector to the trained first decoder D1 and the trained second decoder D2, it is possible to predict the crystal structure represented by the target vector, for example.

In a case where the target vector is a vector representing desired physical properties as described above, for example, field data corresponding to the vector is output. Because a plurality of query points is input to the trained second decoder D2 and the trained second decoder D2 to obtain the field data at the plurality of query points, it is also possible to produce a crystal structure of a substance having the desired physical properties on the basis of the field data.

As the target vector, measurement data of X-ray diffraction (XRD) can also be used, for example. In this case, the XRD measurement data of the target substance is input to the trained first decoder D1 and the trained second decoder D2. Thus, the crystal structure of the target substance can be predicted. Furthermore, the trained first decoder D1 and the trained second decoder D2 of the present embodiment can also be used in combination with an encoder that processes some other modal such as text data.

<Operations of Information Processing Device 10 >

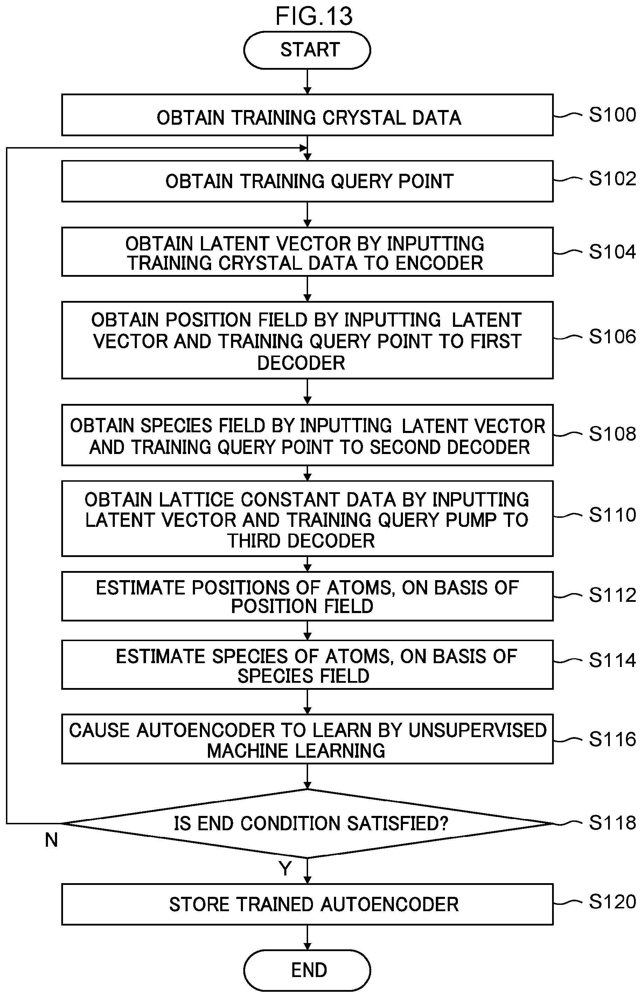

Next, operations of the information processing device 10 according to the embodiment are described with reference to the drawings. Receiving the training crystal data, the information processing device 10 stores the training crystal data into the data storage unit 100. The information processing device 10 then receives an instruction signal to start a training process, and executes a trained model generation process routine shown in Fig. 13.

<Trained Model Generation Process Routine>

In step S100, the training acquisition unit 102 acquires a plurality of sets of the training crystal data stored in the data storage unit 100.

In step S102, the training acquisition unit 102 sets one set of the training crystal data from among the plurality of sets of the training crystal data acquired in step S100. The training unit 104 then sets a plurality of training query points $p_i$ in the space in the substance indicated by the set training crystal data. Note that i is an index for identifying the training query points.

In step S104, the training unit 104 obtains the latent vector z by inputting the position data P, the species data S, and the lattice constant data L in the training crystal data set in step S102 to the encoder E of the autoencoder AE.

In step S106, the training unit 104 obtains the position field $f_p$ by inputting the combination of the training query points $p_i$ set in step S102 and the latent vector z set in step S104 to the first decoder D1. Note that the training unit 104 obtains the position field $f_p$ for each query point among the plurality of the training query points.

In step S108, the training unit 104 obtains the species field $f_s$ by inputting the combination of the training query points $p_i$ set in step S102 and the latent vector z set in step S104 to the second decoder D2. Note that the training unit 104 obtains the species field $f_s$ for each query point among the plurality of the training query points.

In step S110, the training unit 104 obtains the lattice constant data $L_e$ by inputting the combination of the training query points $p_i$ set in step S102 and the latent vector z set in step S104 to the third decoder D3. Note that the training unit 104 obtains the lattice constant data $L_e$ for each query point among the plurality of the training query points.

In step S112, the training unit 104 estimates the positions of atoms on the basis of the position field $f_p$ obtained in step S106. Note that the training unit 104 updates the positions of the plurality of the training query points by the above-described method, and thus, obtains the final estimated positions $P_e$.

In step S114, the training unit 104 estimates the species of atoms, on the basis of the estimated positions $P_e$ of atoms obtained in step S112 and the species field $f_s$ obtained in step S108. As a result, the training unit 104 obtains the estimated species $S_e$ of the atoms present at the estimated positions $P_e$.

In step S116, the training unit 104 trains the autoencoder AE using unsupervised machine learning so that the estimated positions $P_e$ of atoms obtained in step S112 match the position data P of atoms in the training crystal data, the estimated species $S_e$ of atoms obtained in step S114 match the species data S of atoms in the training crystal data, and the lattice constant data $L_e$ obtained in step S110 matches the lattice constant data L in the training crystal data, and thus, produces the trained first decoder D1 and the trained second decoder D2.

The process in steps S102 to S116 is repeated until a condition for an end of machine learning are satisfied. As the condition for an end of machine learning, it is possible to adopt a condition such as whether a machine learning process has been repeated a predetermined number of times, or whether the error between the data output from the autoencoder AE and the training crystal data is equal to or smaller than a predetermined threshold, for example. Although a case where one set of the training crystal data is set and training is then performed has been described above as an example, the embodiment is not limited to this, and machine learning may be performed using a plurality of sets of the training crystal data at once.

In step S118, the training unit 104 determines whether the above-described end condition is satisfied. If the end condition is satisfied, the operation proceeds to step S120. If the end condition is not satisfied, on the other hand, the operation returns to step S102.

In step S120, the training unit 104 stores the trained autoencoder AE obtained by the machine learning process in steps S102 to S116, into the trained model storage unit 106.

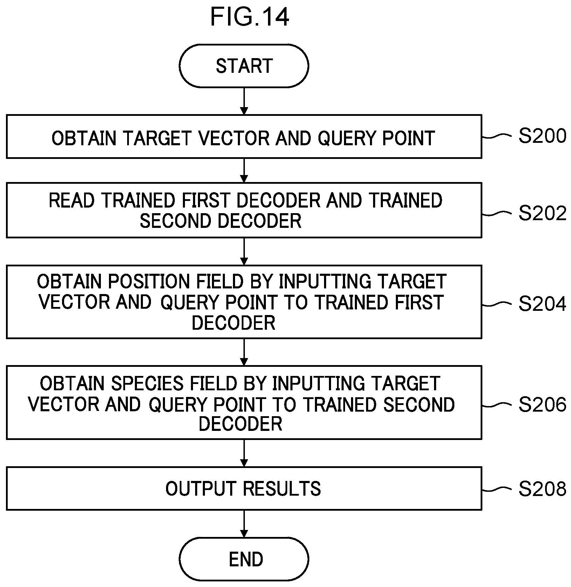

Next, receiving the target vector and a query point, the information processing device 10 performs an estimation process routine shown in Fig. 14.

<Estimation Process Routine>

In step S200, the acquisition unit 108 acquires the target vector and a query point.

In step S202, the processing unit 110 reads the trained first decoder D1 and the trained second decoder D2 stored in the trained model storage unit 106.

In step S204, the processing unit 110 obtains the position field $f_p$ corresponding to the target vector and the query point, by inputting the target vector and the query point acquired in step S200 to the trained first decoder D1 read in step S202.

In step S206, the processing unit 110 obtains the species field $f_s$ corresponding to the target vector and the query point, by inputting the target vector and the query point acquired in step S200 to the trained second decoder D2 read in step S202.

In step S208, the processing unit 110 outputs the position field $f_p$ acquired in step S204 and the species field $f_s$ acquired in step S206 as the results.

As described above, with the information processing device 10, it is possible to train a neural network model expressing a field representing the structure of a substance formed with an atomic point cloud. Also, with the information processing device 10, it is possible to express a field representing the structure of a substance formed with an atomic point cloud, using a neural network model.

Specifically, the information processing device 10 obtains training crystal data that is training crystal data representing the crystal structure of a substance for training and includes position data representing the positions of atoms constituting the crystal of the substance for training, species data representing the species of the atoms constituting the crystal of the substance for training, and lattice constant data of the crystal of the substance for training, and a training query point that is an attention point in the substance for training. When training an autoencoder using unsupervised machine learning, the information processing device 10 obtains a latent vector representing the crystal structure of the substance for training, by inputting the training crystal data to the encoder of the autoencoder. The information processing device 10 inputs the combination of the latent vector and the training query point to the first decoder of the autoencoder, and thus, obtains a position field representing the field of the positions of the atoms constituting the crystal of the substance for training. The information processing device 10 estimates the positions of the atoms constituting the crystal of the substance for training, on the basis of the position field output from the first decoder. The information processing device 10 inputs the combination of the latent vector and the training query point to the second decoder of the autoencoder, and thus, obtains species field data representing the field of the species of the atoms constituting the crystal of the substance for training. The information processing device 10 estimates the species of the atoms constituting the crystal of the substance for training, on the basis of the species field output from the second decoder. The information processing device 10 inputs the combination of the latent vector and the training query point to the third decoder of the autoencoder, and thus, obtains lattice constant data of the crystal of the substance for training. The information processing device 10 trains the autoencoder using unsupervised machine learning so that the combination of the estimated atomic positions, the estimated atomic species, and the lattice constant data output from the third decoder corresponds to the combination of the position data, the species data, and the lattice constant data in the training crystal data, and thus, produces the trained first decoder and the trained second decoder.

In the present embodiment, by adopting the method as described above, a field representing the structure of a substance can be expressed with a neural field. Also, it is possible to generate crystal data from a fixed-length vector.

Also, the information processing device 10 obtains a target vector and a query point that is an attention point in a substance, and acquires field data corresponding to the query point by inputting the target vector and the query point to the first decoder and the second decoder that are an example of the trained neural network model. This field data is indicated by the position field representing the field of the positions of atoms constituting the crystal of the substance and the species field representing the field of the species of atoms constituting the crystal of the substance. When the arbitrary vector in place of the latent vector and the query point are input, the first decoder outputs a position field corresponding to the query point. When the arbitrary vector in place of the latent vector and the query point are input, the second decoder outputs a species field corresponding to the query point. The information processing device 10 obtains the position field corresponding to the query point by inputting the target vector and the query point to the first decoder. The information processing device 10 also obtains the species field corresponding to the query point by inputting the target vector and the query point to the second decoder. In a case where the target vector is a vector representing desired physical properties, for example, field data corresponding to the vector is output. Because a plurality of query points is input to the trained second decoder D2 and the trained second decoder D2 to obtain the field data at the plurality of query points, it is also possible to produce a crystal structure of a substance having the desired physical properties on the basis of the field data.

In a case where the target vector is changed, it is preferable to retrain the trained first decoder D1 and the trained second decoder D2. In this case, the trained first decoder D1 and the trained second decoder D2 are used as preliminary trained models.

Although a case where a field representing a crystal structure is modeled with a neural field has been described as an example in the above embodiment, the embodiment is not limited to this. A crystal structure including iterative structures is not necessarily used, but the structure of a substance formed with an atomic point cloud not including iterative structures may be modeled with a neural field. In this case, estimation of the lattice constant may be unnecessary, and thus, the lattice decorder shown in Fig. 1 may be unnecessary.

Although a case where the vector z input to the decoders is a latent vector output from the encoder has been described as an example in the above embodiment, the embodiment is not limited to this. For example, a vector representing physical properties desired by the user may be z, and the vector z may be given to the decoders (for example, conditions such as bandgap = xxx and formation energy = xxx are expressed by a vector). Alternatively, random noise may be used as the vector z.

Although a case where an autoencoder including an encoder and decoders is used as a neural network model has been described as an example in the above embodiment, the embodiment is not limited to this. As the neural network model, some other model may be used, and, for example, a generative adversarial network (GAN) may be used.

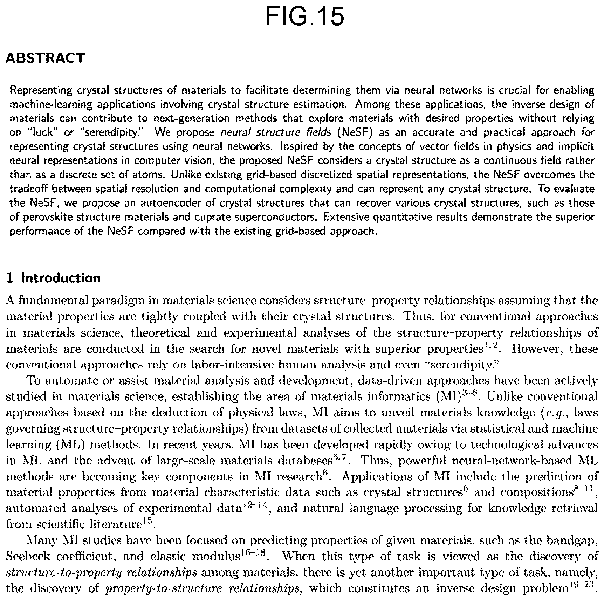

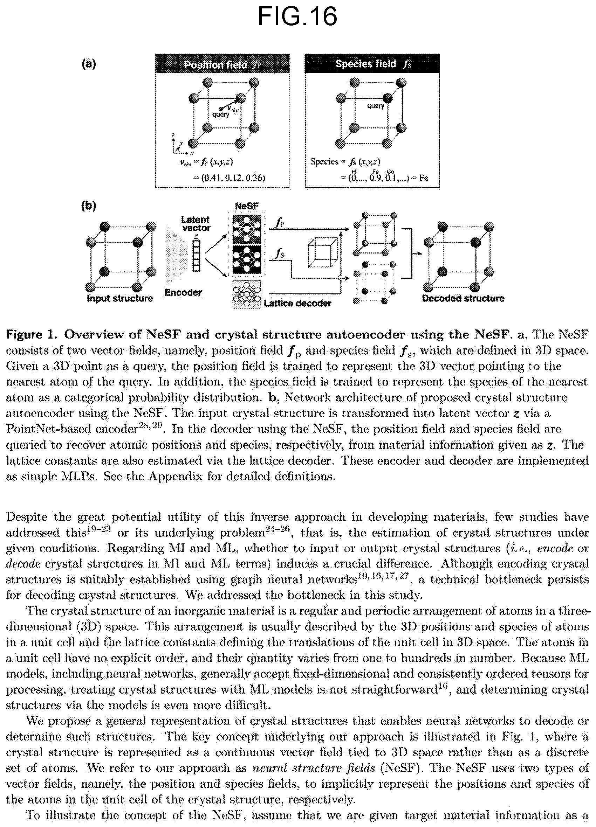

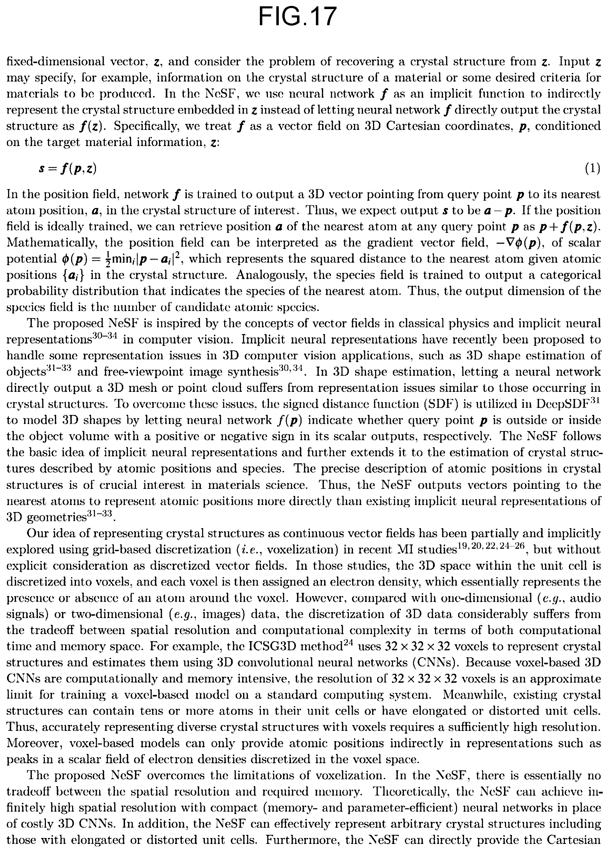

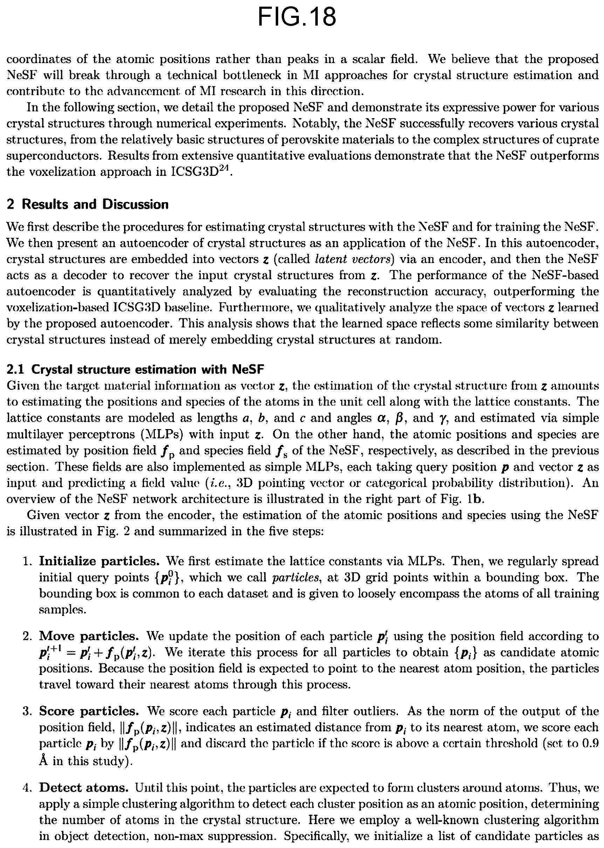

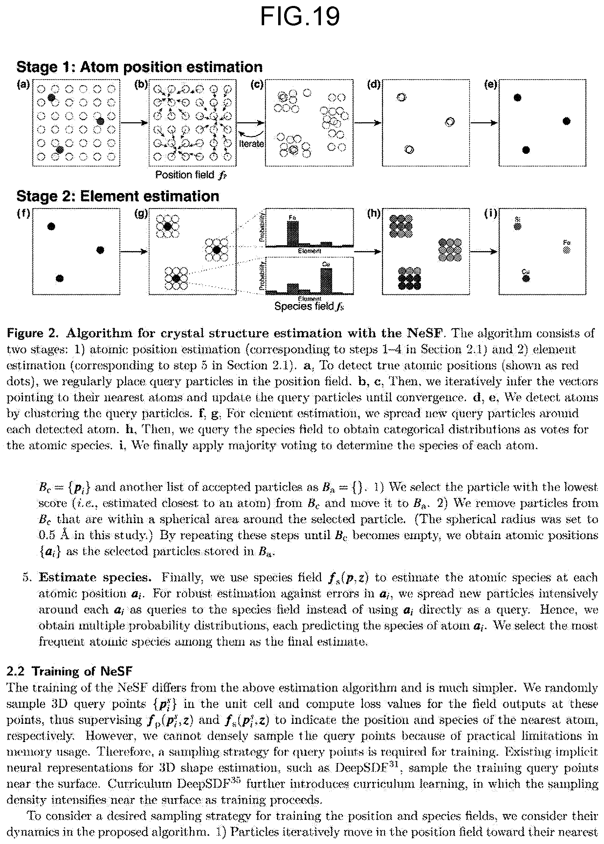

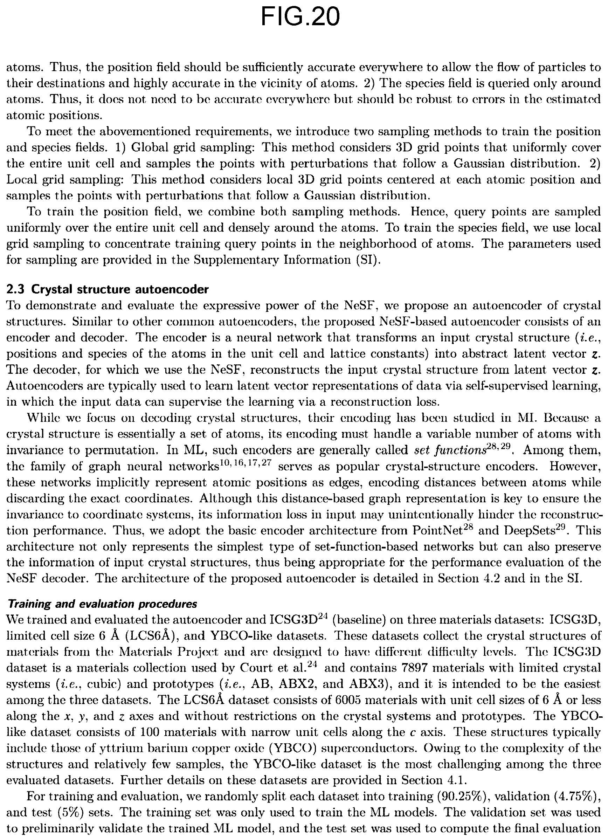

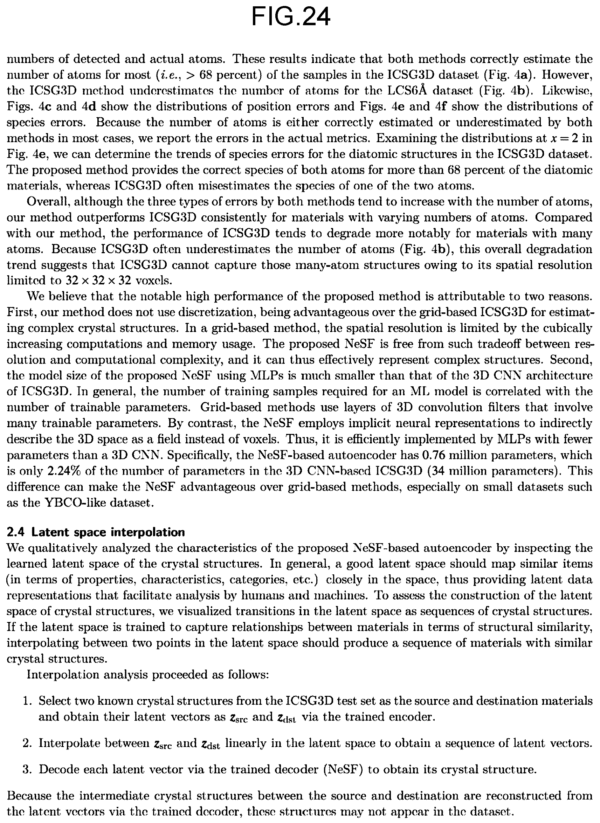

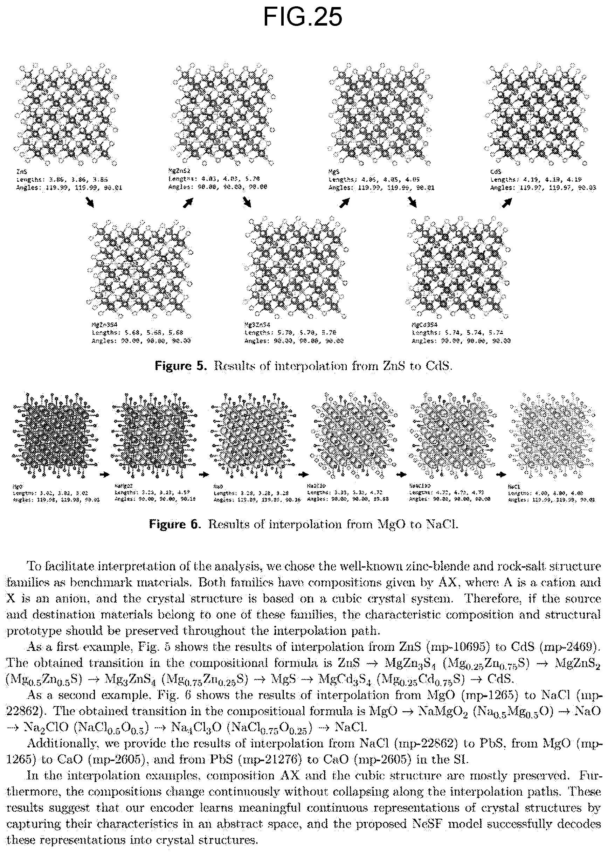

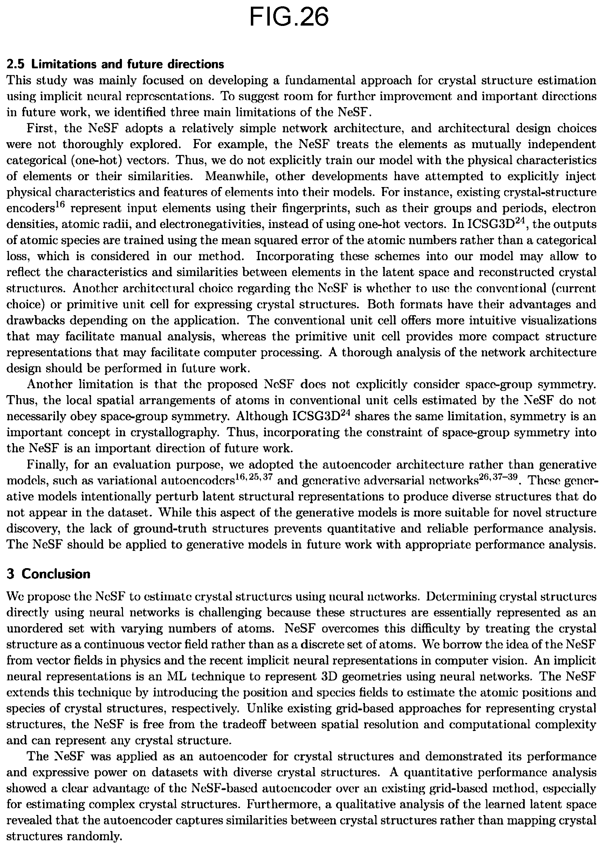

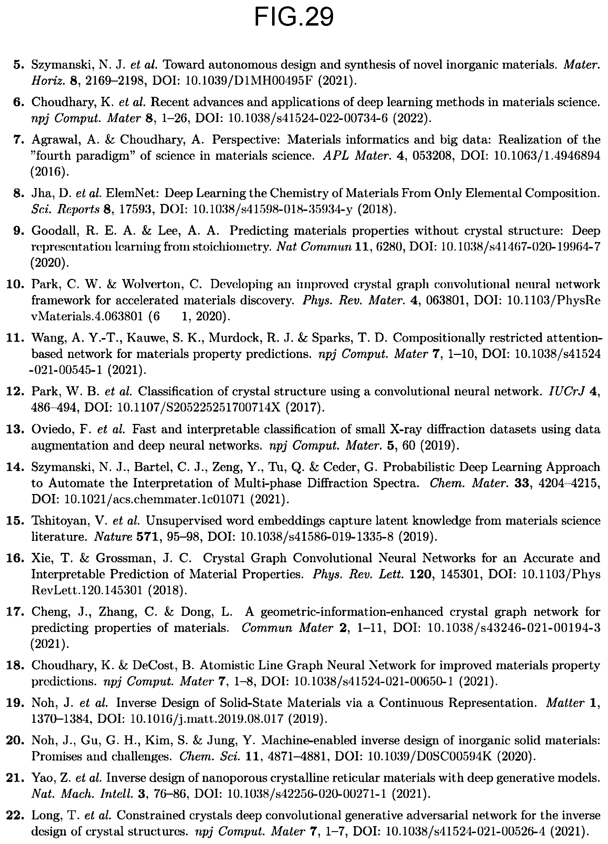

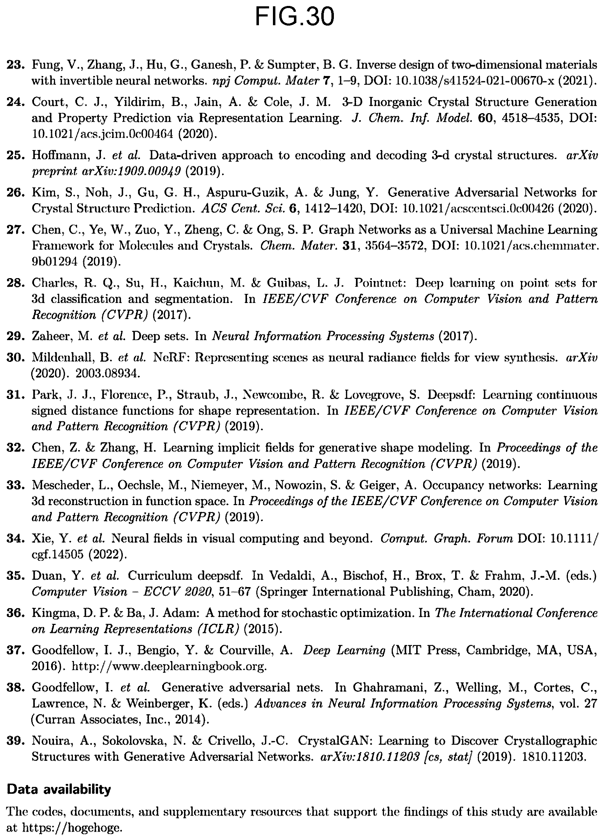

Figs. 15 to 30 are charts for explaining the present embodiment in detail.

Alternatively, the respective processes performed by the CPU reading software (programs) in the above embodiment may be performed by various processors other than a CPU. Examples of the processors in this case include a programmable logic device (PLD) in which the circuit configuration can be changed after manufacturing, such as a field-programmable gate array (FPGA), and a dedicated electric circuit that is a processor having a circuit configuration designed exclusively for performing specific processing, such as an application specific integrated circuit (ASIC). Further, each process may be performed by one of these various processors, or by a combination of two or more processors of the same type or different types (for example, a plurality of FPGAs, or a combination of a CPU and an FPGA). More specifically, the hardware structure of any of these various processors is an electric circuit in which circuit elements such as semiconductor elements are combined.

In the above embodiment, a mode in which each program is stored (installed, for example) beforehand into a storage device has been described, but the embodiment is not limited to this. The programs may be provided in a form stored in a storage medium such as a CD-ROM, a DVD-ROM, a Blu-ray Disc, or a USB memory. Alternatively, the programs may be downloaded from an external device via a network.

(Supplementary Notes)

In the following, modes of the present disclosure are noted.

(Supplementary note 1)

An information processing device including a processing unit that expresses a field representing a structure of a substance formed with an atomic point cloud, using a neural network model.

(Supplementary note 2)

The information processing device of supplementary note 1, further including

an acquisition unit that acquires a target vector and a query point that is an attention point in the substance, in which the neural network model is a trained neural network model that outputs field data representing a field of the structure of the substance at the query point when an arbitrary vector and the query point are input, and
the processing unit obtains the field data corresponding to the query point by inputting the target vector and the query point acquired by the acquisition unit to the trained neural network model.

(Supplementary note 3)

The information processing device of supplementary note 2, in which

the field data is represented by a position field representing a field of positions of atoms constituting crystal of the substance and a species field representing a field of species of atoms constituting the crystal of the substance,

the trained neural network model includes a trained first neural network model and a trained second neural network model,

the trained first neural network model outputs the position field corresponding to the query point when the arbitrary vector and the query point are input,

the trained second neural network model outputs the species field corresponding to the query point when the arbitrary vector and the query point are input, and

the processing unit

obtains the position field corresponding to the query point by inputting the target vector and the query point acquired by the acquisition unit to the trained first neural network model, and

obtains the species field corresponding to the query point by inputting the target vector and the query point acquired by the acquisition unit to the trained second neural network model.

(Supplementary note 4)

The information processing device of supplementary note 2 or 3, in which

the trained neural network model

is a trained model produced beforehand by machine learning on the basis of training crystal data that is crystal data representing a crystal structure of the substance and includes position data representing positions of atoms constituting crystal of the substance, species data representing species of atoms constituting the crystal of the substance, and lattice constant data of the crystal of the substance.

(Supplementary note 5)

The information processing device of supplementary note 3, in which

the trained neural network model is a trained first decoder and a trained second decoder of a trained autoencoder, a trained encoder of the trained autoencoder outputs a latent vector representing the crystal of the substance when crystal data representing a crystal structure of the substance is input, the crystal data including position data representing positions of atoms constituting the crystal of the substance, species data representing species of atoms constituting the crystal of the substance, and lattice constant data of the crystal of the substance,

the trained first decoder outputs the position field corresponding to the query point when the arbitrary vector in place of the latent vector and the query point are input,

the trained second decoder outputs the species field corresponding to the query point when the arbitrary vector in place of the latent vector and the query point are input, and

the trained first decoder and the trained second decoder are trained models obtained by training an autoencoder by unsupervised machine learning on the basis of the crystal data, the autoencoder including an encoder, a first decoder, and a second decoder.

(Supplementary note 5)

The information processing device of supplementary note 4, in which

the autoencoder further includes a third decoder, and

a trained third decoder is a trained model that outputs lattice constant data of the crystal of the substance when the arbitrary vector in place of the latent vector is input.

(Supplementary note 7)

A trained model generation device including a training unit that trains a neural network model expressing a field representing a structure of a substance formed with an atomic point cloud.

(Supplementary note 8)

The trained model generation device of supplementary note 7, further including

a training acquisition unit that acquires training crystal data representing a crystal structure of the substance for training and a training query point that is an attention point in the substance for training, the training crystal data including position data representing positions of atoms constituting crystal of the substance for training, species data representing species of atoms constituting the crystal of the substance for training, and lattice constant data of the crystal of the substance for training, and,

when training an autoencoder using unsupervised machine learning,

the training unit

obtains a latent vector representing the crystal structure of the substance for training, by inputting the training crystal data to an encoder of the autoencoder,

obtains a position field representing a field of the positions of atoms constituting the crystal of the substance for training, by inputting a combination of the latent vector and the training query point to a first decoder of the autoencoder,

estimates the positions of atoms constituting the crystal of the substance for training, on the basis of the position field output from the first decoder,

obtains species field data representing a field of species of atoms constituting the crystal of the substance for training, by inputting the combination of the latent vector and the training query point to a second decoder of the autoencoder,

estimates species of atoms constituting the crystal of the substance for training, on the basis of the species field output from the second decoder,

obtains lattice constant data of the crystal of the substance for training, by inputting the combination of the latent vector and the training query point to a third decoder of the autoencoder,

and produces a trained first decoder and a trained second decoder by training the autoencoder using unsupervised machine learning so that a combination of the estimated positions of atoms, the estimated species of atoms, and the lattice constant data output from the third decoder corresponds to a combination of the position data, the species data, and the lattice constant data in the training crystal data.

(Supplementary note 9)

An information processing method implemented by a computer to perform a process including expressing a field representing a structure of a substance formed with an atomic point cloud, using a neural network model.

(Supplementary note 10)

A trained model generation method implemented by a computer to perform a process including training a neural network model expressing a field representing a structure of a substance formed with an atomic point cloud.

(Supplementary note 11)

An information processing program for causing a computer to perform a process including expressing a field representing a structure of a substance formed with an atomic point cloud, using a neural network model.

(Supplementary note 12)

A trained model generation program for causing a computer to perform a process including training a neural network model expressing a field representing a structure of a substance formed with an atomic point cloud.

The disclosures of Japanese Patent Application No. 2022-186043, filed on November 21, 2022, and Japanese Patent Application No. 2023-130520, filed on August 9, 2023, are incorporated herein by reference in their entirety. All literatures, patent applications, and technical standards mentioned in this specification are incorporated herein by reference to the same extent as that in a case where each literature, each patent application, and each technical standard are specifically and individually mentioned to be incorporated by reference.

**Claims**

1. An information processing device comprising a processing unit that expresses a field representing a structure of a substance formed with an atomic point cloud, using a neural network model.

2. The information processing device according to claim 1, further comprising

   an acquisition unit that acquires a target vector and a query point that is an attention point in the substance, wherein

the neural network model is a trained neural network model that outputs field data representing a field of the structure of the substance at the query point when an arbitrary vector and the query point are input, and the processing unit obtains the field data corresponding to the query point by inputting the target vector and the query point acquired by the acquisition unit to the trained neural network model.

3. The information processing device according to claim 2, wherein

the field data is represented by a position field representing a field of a position of an atom forming crystal of the substance and a species field representing a field of a species of the atom forming the crystal of the substance, the trained neural network model includes a trained first neural network model and a trained second neural network model,

the trained first neural network model outputs the position field corresponding to the query point when the arbitrary vector and the query point are input,

the trained second neural network model outputs the species field corresponding to the query point when the arbitrary vector and the query point are input, and

the processing unit

obtains the position field corresponding to the query point by inputting the target vector and the query point acquired by the acquisition unit to the trained first neural network model, and

obtains the species field corresponding to the query point by inputting the target vector and the query point acquired by the acquisition unit to the trained second neural network model.

4. The information processing device according to claim 2 or 3, wherein

the trained neural network model

is a trained model produced beforehand by machine learning on a basis of training crystal data that is crystal data representing a crystal structure of the substance and includes position data representing a position of an atom forming crystal of the substance, species data representing a species of the atom forming the crystal of the substance, and lattice constant data of the crystal of the substance.

5. The information processing device according to claim 3, wherein

the trained neural network model is a trained first decoder and a trained second decoder of a trained autoencoder, a trained encoder of the trained autoencoder outputs a latent vector representing the crystal of the substance when crystal data representing a crystal structure of the substance is input, the crystal data including position data representing a position of an atom forming the crystal of the substance, species data representing a species of the atom forming the crystal of the substance, and lattice constant data of the crystal of the substance,

the trained first decoder outputs the position field corresponding to the query point when the arbitrary vector in place of the latent vector and the query point are input,

the trained second decoder outputs the species field corresponding to the query point when the arbitrary vector in place of the latent vector and the query point are input, and

the trained first decoder and the trained second decoder are trained models obtained by training an autoencoder by unsupervised machine learning on a basis of the crystal data, the autoencoder including an encoder, a first decoder, and a second decoder.

6. The information processing device according to claim 5, wherein

the autoencoder further includes a third decoder, and

a trained third decoder is a trained model that outputs lattice constant data of the crystal of the substance when the arbitrary vector in place of the latent vector is input.

7. A trained model generation device comprising a training unit that trains a neural network model expressing a field representing a structure of a substance formed with an atomic point cloud.

8. The trained model generation device according to claim 7, further comprising

a training acquisition unit that acquires training crystal data representing a crystal structure of the substance for training and a training query point that is an attention point in the substance for training, the training crystal data including position data representing a position of an atom forming crystal of the substance for training, species data representing a species of the atom forming the crystal of the substance for training, and lattice constant data of the crystal of the substance for training, and,

when training an autoencoder using unsupervised machine learning,

the training unit

obtains a latent vector representing the crystal structure of the substance for training, by inputting the training crystal data to an encoder of the autoencoder,

obtains a position field representing a field of a position of an atom forming the crystal of the substance for training, by inputting a combination of the latent vector and the training query point to a first decoder of the autoencoder,

estimates the position of the atom forming the crystal of the substance for training, on a basis of the position field output from the first decoder,

obtains species field representing a field of a species of the atom forming the crystal of the substance for training, by inputting the combination of the latent vector and the training query point to a second decoder of the autoencoder,

estimates the species of the atom forming the crystal of the substance for training, on a basis of the species field output from the second decoder,

obtains lattice constant data of the crystal of the substance for training, by inputting the combination of the latent vector and the training query point to a third decoder of the autoencoder,

and produces a trained first decoder and a trained second decoder by training the autoencoder using unsupervised machine learning so that a combination of the estimated position of the atom, the estimated species of the atom, and the lattice constant data output from the third decoder corresponds to a combination of the position data, the species data, and the lattice constant data in the training crystal data.

9. An information processing method implemented by a computer to perform a process including expressing a field representing a structure of a substance formed with an atomic point cloud, using a neural network model.

10. A trained model generation method implemented by a computer to perform a process including training a neural network model expressing a field representing a structure of a substance formed with an atomic point cloud.

11. An information processing program for causing a computer to perform a process including expressing a field representing a structure of a substance formed with an atomic point cloud, using a neural network model.

12. A trained model generation program for causing a computer to perform a process including training a neural network model expressing a field representing a structure of a substance formed with an atomic point cloud.

FIG.1

**(a)**

Position field $f_P$

$$v_{dkr} = f_P(x,y,z)$$

$$= (0.41, 0.12, 0.36)$$

Species field $f_S$

query

$$Species = f_S(x,y,z)$$

$$= (0,\overset{H}{...}, 0.9, 0.1,...) = Fe$$

**(b)**

Input structure — Encoder — Latent vector $\tilde{z}$ — NeSF — $f_P$ / $f_S$ — Lattice decoder — Decoded structure

FIG.2

## Stage 1: Atom position estimation

Position field $f_P$

## Stage 2: Element estimation

Species field $f_S$

# FIG.3

a

$Tm_3TlC$
mp-22556
(ICSG3D dataset)

- C
- Tm
- Tl
- Gd

b

$MnInF_3$
mp-998241
(LSC6Å dataset)

- F
- Mn
- In
- Zr

c

$ErBa_2(Cu_{0.33}Zn_{0.67})_3O_7$
mp-1214708
(YBCO-like dataset)

- O
- Zn
- Cu
- Lu
- Er
- Ba

Input        Proposed        ICSG3D

# FIG.4

(a)

Signed Error of Number of Atoms

(b)

Signed Error of Number of Atoms

(c)

Position Error (Actual)

(d)

Position Error (Actual)

(e)

Species Error (Actual)

(f)

Species Error (Actual)

ICSG3D dataset

LCS6Å dataset

# FIG.5

Figure 5. Results of interpolation from ZnS to CdS.

# FIG.6

**Figure 6.** Results of interpolation from MgO to NaCl.

# FIG.7

# FIG.8

10

| TRAINING ACQUISITION UNIT | ~ 102 |

| DATA STORAGE UNIT | ~ 100 |

| TRAINING UNIT | ~ 104 |

| ACQUISITION UNIT | ~ 108 |

| TRAINED MODEL STORAGE UNIT | ~ 106 |

| PROCESSING UNIT | ~ 110 |

FIG.9

| CRYSTAL ID | POSITION DATA | SPECIES DATA | LATTICE CONSTANT DATA |
|---|---|---|---|
| 00001 | P1 | S1 | L1 |
| 00002 | P2 | S2 | L2 |
| 00003 | . . . | . . . | . . . |
| . . . | . . . | . . . | . . . |

# FIG.10

FIG.11

Position field $f_p$

FIG.12

## FIG.13

```
        ┌─────────────┐
        │    START    │
        └──────┬──────┘
               ↓
```

| | |
|---|---|
| OBTAIN TRAINING CRYSTAL DATA | S100 |
| OBTAIN TRAINING QUERY POINT | S102 |
| OBTAIN LATENT VECTOR BY INPUTTING TRAINING CRYSTAL DATA TO ENCODER | S104 |
| OBTAIN POSITION FIELD BY INPUTTING LATENT VECTOR AND TRAINING QUERY POINT TO FIRST DECODER | S106 |
| OBTAIN SPECIES FIELD BY INPUTTING LATENT VECTOR AND TRAINING QUERY POINT TO SECOND DECODER | S108 |
| OBTAIN LATTICE CONSTANT DATA BY INPUTTING LATENT VECTOR AND TRAINING QUERY PUMP TO THIRD DECODER | S110 |
| ESTIMATE POSITIONS OF ATOMS, ON BASIS OF POSITION FIELD | S112 |
| ESTIMATE SPECIES OF ATOMS, ON BASIS OF SPECIES FIELD | S114 |
| CAUSE AUTOENCODER TO LEARN BY UNSUPERVISED MACHINE LEARNING | S116 |

IS END CONDITION SATISFIED? — S118

N

Y

| | |
|---|---|
| STORE TRAINED AUTOENCODER | S120 |

```
        ┌─────────────┐
        │     END     │
        └─────────────┘
```

# FIG.14

START

OBTAIN TARGET VECTOR AND QUERY POINT — S200

READ TRAINED FIRST DECODER AND TRAINED SECOND DECODER — S202

OBTAIN POSITION FIELD BY INPUTTING TARGET VECTOR AND QUERY POINT TO TRAINED FIRST DECODER — S204

OBTAIN SPECIES FIELD BY INPUTTING TARGET VECTOR AND QUERY POINT TO TRAINED SECOND DECODER — S206

OUTPUT RESULTS — S208

END

# FIG.15

## ABSTRACT

Representing crystal structures of materials to facilitate determining them via neural networks is crucial for enabling machine-learning applications involving crystal structure estimation. Among these applications, the inverse design of materials can contribute to next-generation methods that explore materials with desired properties without relying on "luck" or "serendipity." We propose *neural structure fields* (NeSF) as an accurate and practical approach for representing crystal structures using neural networks. Inspired by the concepts of vector fields in physics and implicit neural representations in computer vision, the proposed NeSF considers a crystal structure as a continuous field rather than as a discrete set of atoms. Unlike existing grid-based discretized spatial representations, the NeSF overcomes the tradeoff between spatial resolution and computational complexity and can represent any crystal structure. To evaluate the NeSF, we propose an autoencoder of crystal structures that can recover various crystal structures, such as those of perovskite structure materials and cuprate superconductors. Extensive quantitative results demonstrate the superior performance of the NeSF compared with the existing grid-based approach.

## 1 Introduction

A fundamental paradigm in materials science considers structure–property relationships assuming that the material properties are tightly coupled with their crystal structures. Thus, for conventional approaches in materials science, theoretical and experimental analyses of the structure–property relationships of materials are conducted in the search for novel materials with superior properties[1,2]. However, these conventional approaches rely on labor-intensive human analysis and even "serendipity."

To automate or assist material analysis and development, data-driven approaches have been actively studied in materials science, establishing the area of materials informatics (MI)[3–6]. Unlike conventional approaches based on the deduction of physical laws, MI aims to unveil materials knowledge (*e.g.*, laws governing structure–property relationships) from datasets of collected materials via statistical and machine learning (ML) methods. In recent years, MI has been developed rapidly owing to technological advances in ML and the advent of large-scale materials databases[6,7]. Thus, powerful neural-network-based ML methods are becoming key components in MI research[6]. Applications of MI include the prediction of material properties from material characteristic data such as crystal structures[6] and compositions[8–11], automated analyses of experimental data[12–14], and natural language processing for knowledge retrieval from scientific literature[15].

Many MI studies have been focused on predicting properties of given materials, such as the bandgap, Seebeck coefficient, and elastic modulus[16–18]. When this type of task is viewed as the discovery of *structure-to-property relationships* among materials, there is yet another important type of task, namely, the discovery of *property-to-structure relationships*, which constitutes an inverse design problem[19–23].

# FIG.16

**(a)**

**(b)**

Figure 1. Overview of NeSF and crystal structure autoencoder using the NeSF. a, The NeSF consists of two vector fields, namely, position field $f_p$ and species field $f_s$, which are defined in 3D space. Given a 3D point as a query, the position field is trained to represent the 3D vector pointing to the nearest atom of the query. In addition, the species field is trained to represent the species of the nearest atom as a categorical probability distribution. b, Network architecture of proposed crystal structure autoencoder using the NeSF. The input crystal structure is transformed into latent vector $z$ via a PointNet-based encoder[28,29]. In the decoder using the NeSF, the position field and species field are queried to recover atomic positions and species, respectively, from material information given as $z$. The lattice constants are also estimated via the lattice decoder. These encoder and decoder are implemented as simple MLPs. See the Appendix for detailed definitions.

Despite the great potential utility of this inverse approach in developing materials, few studies have addressed this[19–23] or its underlying problem[24–26], that is, the estimation of crystal structures under given conditions. Regarding MI and ML, whether to input or output crystal structures (i.e., *encode* or *decode* crystal structures in MI and ML terms) induces a crucial difference. Although encoding crystal structures is suitably established using graph neural networks[10,16,17,27], a technical bottleneck persists for decoding crystal structures. We addressed the bottleneck in this study.

The crystal structure of an inorganic material is a regular and periodic arrangement of atoms in a three-dimensional (3D) space. This arrangement is usually described by the 3D positions and species of atoms in a unit cell and the lattice constants defining the translations of the unit cell in 3D space. The atoms in a unit cell have no explicit order, and their quantity varies from one to hundreds in number. Because ML models, including neural networks, generally accept fixed-dimensional and consistently ordered tensors for processing, treating crystal structures with ML models is not straightforward[16], and determining crystal structures via the models is even more difficult.

We propose a general representation of crystal structures that enables neural networks to decode or determine such structures. The key concept underlying our approach is illustrated in Fig. 1, where a crystal structure is represented as a continuous vector field tied to 3D space rather than as a discrete set of atoms. We refer to our approach as *neural structure fields* (NeSF). The NeSF uses two types of vector fields, namely, the position and species fields, to implicitly represent the positions and species of the atoms in the unit cell of the crystal structure, respectively.

To illustrate the concept of the NeSF, assume that we are given target material information as a

# FIG.17

fixed-dimensional vector, $z$, and consider the problem of recovering a crystal structure from $z$. Input $z$ may specify, for example, information on the crystal structure of a material or some desired criteria for materials to be produced. In the NeSF, we use neural network $f$ as an implicit function to indirectly represent the crystal structure embedded in $z$ instead of letting neural network $f$ directly output the crystal structure as $f(z)$. Specifically, we treat $f$ as a vector field on 3D Cartesian coordinates, $p$, conditioned on the target material information, $z$:

$$s = f(p,z) \tag{1}$$

In the position field, network $f$ is trained to output a 3D vector pointing from query point $p$ to its nearest atom position, $a$, in the crystal structure of interest. Thus, we expect output $s$ to be $a - p$. If the position field is ideally trained, we can retrieve position $a$ of the nearest atom at any query point $p$ as $p + f(p,z)$. Mathematically, the position field can be interpreted as the gradient vector field, $-\nabla\phi(p)$, of scalar potential $\phi(p) = \frac{1}{2}\min_i|p - a_i|^2$, which represents the squared distance to the nearest atom given atomic positions $\{a_i\}$ in the crystal structure. Analogously, the species field is trained to output a categorical probability distribution that indicates the species of the nearest atom. Thus, the output dimension of the species field is the number of candidate atomic species.

The proposed NeSF is inspired by the concepts of vector fields in classical physics and implicit neural representations[30-34] in computer vision. Implicit neural representations have recently been proposed to handle some representation issues in 3D computer vision applications, such as 3D shape estimation of objects[31-33] and free-viewpoint image synthesis[30,34]. In 3D shape estimation, letting a neural network directly output a 3D mesh or point cloud suffers from representation issues similar to those occurring in crystal structures. To overcome these issues, the signed distance function (SDF) is utilized in DeepSDF[31] to model 3D shapes by letting neural network $f(p)$ indicate whether query point $p$ is outside or inside the object volume with a positive or negative sign in its scalar outputs, respectively. The NeSF follows the basic idea of implicit neural representations and further extends it to the estimation of crystal structures described by atomic positions and species. The precise description of atomic positions in crystal structures is of crucial interest in materials science. Thus, the NeSF outputs vectors pointing to the nearest atoms to represent atomic positions more directly than existing implicit neural representations of 3D geometries[31-33].

Our idea of representing crystal structures as continuous vector fields has been partially and implicitly explored using grid-based discretization (i.e., voxelization) in recent MI studies[19,20,22,24-26], but without explicit consideration as discretized vector fields. In those studies, the 3D space within the unit cell is discretized into voxels, and each voxel is then assigned an electron density, which essentially represents the presence or absence of an atom around the voxel. However, compared with one-dimensional (e.g., audio signals) or two-dimensional (e.g., images) data, the discretization of 3D data considerably suffers from the tradeoff between spatial resolution and computational complexity in terms of both computational time and memory space. For example, the ICSG3D method[24] uses $32 \times 32 \times 32$ voxels to represent crystal structures and estimates them using 3D convolutional neural networks (CNNs). Because voxel-based 3D CNNs are computationally and memory intensive, the resolution of $32 \times 32 \times 32$ voxels is an approximate limit for training a voxel-based model on a standard computing system. Meanwhile, existing crystal structures can contain tens or more atoms in their unit cells or have elongated or distorted unit cells. Thus, accurately representing diverse crystal structures with voxels requires a sufficiently high resolution. Moreover, voxel-based models can only provide atomic positions indirectly in representations such as peaks in a scalar field of electron densities discretized in the voxel space.

The proposed NeSF overcomes the limitations of voxelization. In the NeSF, there is essentially no tradeoff between the spatial resolution and required memory. Theoretically, the NeSF can achieve infinitely high spatial resolution with compact (memory- and parameter-efficient) neural networks in place of costly 3D CNNs. In addition, the NeSF can effectively represent arbitrary crystal structures including those with elongated or distorted unit cells. Furthermore, the NeSF can directly provide the Cartesian

# FIG.18

coordinates of the atomic positions rather than peaks in a scalar field. We believe that the proposed NeSF will break through a technical bottleneck in MI approaches for crystal structure estimation and contribute to the advancement of MI research in this direction.

In the following section, we detail the proposed NeSF and demonstrate its expressive power for various crystal structures through numerical experiments. Notably, the NeSF successfully recovers various crystal structures, from the relatively basic structures of perovskite materials to the complex structures of cuprate superconductors. Results from extensive quantitative evaluations demonstrate that the NeSF outperforms the voxelization approach in ICSG3D[24].

## 2 Results and Discussion

We first describe the procedures for estimating crystal structures with the NeSF and for training the NeSF. We then present an autoencoder of crystal structures as an application of the NeSF. In this autoencoder, crystal structures are embedded into vectors $z$ (called *latent vectors*) via an encoder, and then the NeSF acts as a decoder to recover the input crystal structures from $z$. The performance of the NeSF-based autoencoder is quantitatively analyzed by evaluating the reconstruction accuracy, outperforming the voxelization-based ICSG3D baseline. Furthermore, we qualitatively analyze the space of vectors $z$ learned by the proposed autoencoder. This analysis shows that the learned space reflects some similarity between crystal structures instead of merely embedding crystal structures at random.

### 2.1 Crystal structure estimation with NeSF

Given the target material information as vector $z$, the estimation of the crystal structure from $z$ amounts to estimating the positions and species of the atoms in the unit cell along with the lattice constants. The lattice constants are modeled as lengths $a$, $b$, and $c$ and angles $\alpha$, $\beta$, and $\gamma$, and estimated via simple multilayer perceptrons (MLPs) with input $z$. On the other hand, the atomic positions and species are estimated by position field $f_p$ and species field $f_s$ of the NeSF, respectively, as described in the previous section. These fields are also implemented as simple MLPs, each taking query position $p$ and vector $z$ as input and predicting a field value (*i.e.*, 3D pointing vector or categorical probability distribution). An overview of the NeSF network architecture is illustrated in the right part of Fig. 1b.

Given vector $z$ from the encoder, the estimation of the atomic positions and species using the NeSF is illustrated in Fig. 2 and summarized in the five steps:

1. **Initialize particles.** We first estimate the lattice constants via MLPs. Then, we regularly spread initial query points $\{p_i^0\}$, which we call *particles*, at 3D grid points within a bounding box. The bounding box is common to each dataset and is given to loosely encompass the atoms of all training samples.

2. **Move particles.** We update the position of each particle $p_i'$ using the position field according to $p_i^{t+1} = p_i^t + f_p(p_i^t, z)$. We iterate this process for all particles to obtain $\{p_i\}$ as candidate atomic positions. Because the position field is expected to point to the nearest atom position, the particles travel toward their nearest atoms through this process.

3. **Score particles.** We score each particle $p_i$ and filter outliers. As the norm of the output of the position field, $\|f_p(p_i, z)\|$, indicates an estimated distance from $p_i$ to its nearest atom, we score each particle $p_i$ by $\|f_p(p_i, z)\|$ and discard the particle if the score is above a certain threshold (set to 0.9 Å in this study).

4. **Detect atoms.** Until this point, the particles are expected to form clusters around atoms. Thus, we apply a simple clustering algorithm to detect each cluster position as an atomic position, determining the number of atoms in the crystal structure. Here we employ a well-known clustering algorithm in object detection, non-max suppression. Specifically, we initialize a list of candidate particles as

# FIG.19

**Stage 1: Atom position estimation**

Position field $f_p$

**Stage 2: Element estimation**

Species field $f_s$

Figure 2. **Algorithm for crystal structure estimation with the NeSF**. The algorithm consists of two stages: 1) atomic position estimation (corresponding to steps 1–4 in Section 2.1) and 2) element estimation (corresponding to step 5 in Section 2.1). **a**, To detect true atomic positions (shown as red dots), we regularly place query particles in the position field. **b**, **c**, Then, we iteratively infer the vectors pointing to their nearest atoms and update the query particles until convergence. **d**, **e**, We detect atoms by clustering the query particles. **f**, **g**, For element estimation, we spread new query particles around each detected atom. **h**, Then, we query the species field to obtain categorical distributions as votes for the atomic species. **i**, We finally apply majority voting to determine the species of each atom.

$B_c = \{p_i\}$ and another list of accepted particles as $B_a = \{\}$. 1) We select the particle with the lowest score (*i.e.*, estimated closest to an atom) from $B_c$ and move it to $B_a$. 2) We remove particles from $B_c$ that are within a spherical area around the selected particle. (The spherical radius was set to 0.5 Å in this study.) By repeating these steps until $B_c$ becomes empty, we obtain atomic positions $\{a_i\}$ as the selected particles stored in $B_a$.

5. **Estimate species.** Finally, we use species field $f_s(p, z)$ to estimate the atomic species at each atomic position $a_i$. For robust estimation against errors in $a_i$, we spread new particles intensively around each $a_i$ as queries to the species field instead of using $a_i$ directly as a query. Hence, we obtain multiple probability distributions, each predicting the species of atom $a_i$. We select the most frequent atomic species among them as the final estimate.

## 2.2 Training of NeSF

The training of the NeSF differs from the above estimation algorithm and is much simpler. We randomly sample 3D query points $\{p_i^s\}$ in the unit cell and compute loss values for the field outputs at these points, thus supervising $f_p(p_i^s, z)$ and $f_s(p_i^s, z)$ to indicate the position and species of the nearest atom, respectively. However, we cannot densely sample the query points because of practical limitations in memory usage. Therefore, a sampling strategy for query points is required for training. Existing implicit neural representations for 3D shape estimation, such as DeepSDF[31], sample the training query points near the surface. Curriculum DeepSDF[35] further introduces curriculum learning, in which the sampling density intensifies near the surface as training proceeds.

To consider a desired sampling strategy for training the position and species fields, we consider their dynamics in the proposed algorithm. 1) Particles iteratively move in the position field toward their nearest

# FIG.20

atoms. Thus, the position field should be sufficiently accurate everywhere to allow the flow of particles to their destinations and highly accurate in the vicinity of atoms. 2) The species field is queried only around atoms. Thus, it does not need to be accurate everywhere but should be robust to errors in the estimated atomic positions.

To meet the abovementioned requirements, we introduce two sampling methods to train the position and species fields. 1) Global grid sampling: This method considers 3D grid points that uniformly cover the entire unit cell and samples the points with perturbations that follow a Gaussian distribution. 2) Local grid sampling: This method considers local 3D grid points centered at each atomic position and samples the points with perturbations that follow a Gaussian distribution.

To train the position field, we combine both sampling methods. Hence, query points are sampled uniformly over the entire unit cell and densely around the atoms. To train the species field, we use local grid sampling to concentrate training query points in the neighborhood of atoms. The parameters used for sampling are provided in the Supplementary Information (SI).

## 2.3 Crystal structure autoencoder

To demonstrate and evaluate the expressive power of the NeSF, we propose an autoencoder of crystal structures. Similar to other common autoencoders, the proposed NeSF-based autoencoder consists of an encoder and decoder. The encoder is a neural network that transforms an input crystal structure (*i.e.*, positions and species of the atoms in the unit cell and lattice constants) into abstract latent vector $z$. The decoder, for which we use the NeSF, reconstructs the input crystal structure from latent vector $z$. Autoencoders are typically used to learn latent vector representations of data via self-supervised learning, in which the input data can supervise the learning via a reconstruction loss.

While we focus on decoding crystal structures, their encoding has been studied in MI. Because a crystal structure is essentially a set of atoms, its encoding must handle a variable number of atoms with invariance to permutation. In ML, such encoders are generally called *set functions*[28,29]. Among them, the family of graph neural networks[10,16,17,27] serves as popular crystal-structure encoders. However, these networks implicitly represent atomic positions as edges, encoding distances between atoms while discarding the exact coordinates. Although this distance-based graph representation is key to ensure the invariance to coordinate systems, its information loss in input may unintentionally hinder the reconstruction performance. Thus, we adopt the basic encoder architecture from PointNet[28] and DeepSets[29]. This architecture not only represents the simplest type of set-function-based networks but can also preserve the information of input crystal structures, thus being appropriate for the performance evaluation of the NeSF decoder. The architecture of the proposed autoencoder is detailed in Section 4.2 and in the SI.

### Training and evaluation procedures

We trained and evaluated the autoencoder and ICSG3D[24] (baseline) on three materials datasets: ICSG3D, limited cell size 6 Å (LCS6Å), and YBCO-like datasets. These datasets collect the crystal structures of materials from the Materials Project and are designed to have different difficulty levels. The ICSG3D dataset is a materials collection used by Court et al.[24] and contains 7897 materials with limited crystal systems (*i.e.*, cubic) and prototypes (*i.e.*, AB, ABX2, and ABX3), and it is intended to be the easiest among the three datasets. The LCS6Å dataset consists of 6005 materials with unit cell sizes of 6 Å or less along the $x$, $y$, and $z$ axes and without restrictions on the crystal systems and prototypes. The YBCO-like dataset consists of 100 materials with narrow unit cells along the $c$ axis. These structures typically include those of yttrium barium copper oxide (YBCO) superconductors. Owing to the complexity of the structures and relatively few samples, the YBCO-like dataset is the most challenging among the three evaluated datasets. Further details on these datasets are provided in Section 4.1.

For training and evaluation, we randomly split each dataset into training (90.25%), validation (4.75%), and test (5%) sets. The training set was only used to train the ML models. The validation set was used to preliminarily validate the trained ML model, and the test set was used to compute the final evaluation

# FIG.21

**Table 1. Reconstruction results**

| Method | Proposed | ICSG3D | Proposed | ICSG3D | Proposed | ICSG3D |
|---|---|---|---|---|---|---|
| Dataset | ICSG3D | | LCS6Å | | YBCO-like | |
| Error in number of atoms [%] | 0.53 ± 0.25 | 2.67 ± 0.84 | 6.35 ± 0.50 | 17.28 ± 1.13 | 12.00 | 91.00 |
| Position error (actual) [Å] | 0.0308 ± 0.0112 | 0.0877 ± 0.0306 | 0.1161 ± 0.0073 | 0.2006 ± 0.0132 | 0.2631 | (0.6311) |
| Position error (detected) [Å] | 0.0359 ± 0.0226 | 0.1057 ± 0.0284 | 0.1632 ± 0.0123 | 0.2886 ± 0.0159 | 0.2448 | 0.4358 |
| Species error (actual) [%] | 4.31 ± 0.39 | 64.39 ± 1.91 | 14.78 ± 0.81 | 55.70 ± 1.40 | 14.78 | 78.08 |
| Species error (detected) [%] | 4.36 ± 0.39 | 65.05 ± 1.85 | 16.11 ± 0.66 | 58.32 ± 1.45 | 19.89 | 54.24 |

We compared the performance of the proposed NeSF-based crystal structure autoencoder and ICSG3D method for crystal structure reconstruction on three datasets (ICSG3D, LCS6Å, and YBCO-like). The position and species errors were evaluated in two ways based on the actual or detected atomic sites. For the YBCO-like dataset, ICSG3D failed to output any atom for 44 out of the 100 materials and provided no valid score for the position error (actual). We computed a score by excluding those incorrectly estimated materials (shown in parentheses), indicating that the actual performance of ICSG3D is worse the displayed value.

scores after training, validation, and hyperparameter tuning. The hyperparameters were tuned based on the validation scores from the LCS6Å dataset. To reduce the performance variation owing to randomness (*e.g.*, randomness in initialization of network weights), we repeated training and evaluation 10 times with different random seeds and evaluated the performance using the mean and standard deviation of the scores. Because the YBCO-like dataset has only 100 samples, it was treated slightly differently from the other two evaluated datasets. To reduce the performance variation owing to data splitting, we adopted twentyfold cross-validation for the YBCO-like dataset but evaluated it once instead of 10 times. The iterative training of the neural networks was conducted using stochastic gradient descent with Adam[36] as the optimizer. Detailed training procedures, including the loss function definition, are provided in Section 4.3.

The reconstruction performance was measured in terms of errors in the number of atoms, position, and species. The error in the number of atoms is the rate of materials for which the number of atoms in the unit cell is incorrectly estimated. The position error is the average error of the reconstructed atomic positions. Depending on the denominator of the metric, we evaluated the position error in two ways. An *actual* metric was used to evaluate the mean position errors at the actual atomic sites of the crystal structure by computing their shortest distances to estimated atomic sites. By contrast, a *detected* metric was used to evaluate the errors at the estimated sites by computing their shortest distances to the actual atomic sites. The actual metric is more sensitive to errors related to underestimation of the number of atoms, whereas the detected metric is more sensitive to errors related to overestimation. The species error is the average rate of atoms with incorrectly estimated species. Analogous to the position error, the species error was evaluated using actual and detected metrics. Lower values of these metrics indicate better performance.

### *Quantitative performance comparisons with ICSG3D*

Table 1 lists the reconstruction errors of the proposed NeSF-based autoencoder and ICSG3D baseline on the test sets of the three datasets. Overall, the proposed method consistently outperforms ICSG3D in all the evaluation metrics, with substantial performance improvements for the species error in all datasets and for all the metrics on the YBCO-like dataset. Fig. 3 shows crystal structures from the three evaluated datasets, comparing test samples and reconstruction results by the proposed autoencoder and ICSG3D.

For the ICSG3D dataset, which is the easiest of the three datasets, ICSG3D achieves good performance for the error in number of atoms and position error, but provides high species error (approximately 65% in both actual and detected metrics). By contrast, the proposed method achieves slightly better position error and error in number of atoms and drastically lower species error (approximately 4%). We believe that this is because ICSG3D estimates the atomic species via electron density maps, whereas the proposed method more directly represents atomic species as categorical distributions. Extending ICSG3D

# FIG.22

**Figure 3. Reconstruction examples.** For the three evaluated datasets (rows), we show test samples (first column) and reconstruction results by the proposed (second column) and ICSG3D (third column) methods. The full results are available online.

to estimate a categorical distribution at each voxel is impractical because it would require approximately $100 \times 32^3$ times the memory usage in the output (i.e., requiring 100 species categories for each of the $32^3$ voxels in addition to one electron density map).

For the the LCS6Å and YBCO-like datasets, which are more challenging than the ICSG3D dataset, the performance advantage of the proposed method is even clearer, especially regarding the position error. The LCS6Å dataset contains a variety of crystal structures (e.g., non-cubic structures, distorted crystal structures), whereas the YBCO-like dataset contains very narrow crystal structures. In addition, the YBCO-like dataset contains few samples, possibly leading to model overfitting (i.e., the performance on the test set is likely to degrade significantly). Despite these difficulties, the proposed method can accurately estimate the atomic positions and species.

For a detailed analysis of the relationship between the method performance and structural complexity, Fig. 4 shows the distributions of reconstruction errors according to number of atoms given as medians (points) and 68-percent ranges around them (colored regions) over 10 test trials on materials from the ICSG3D and LCS6Å datasets. The YBCO-like dataset is excluded from this analysis because it contains only materials with 13 atoms in their unit cells. Figs. 4a and 4b show the signed errors between the

# FIG.23

**Figure 4. Relationships between reconstruction performance and number of atoms in conventional unit cell.** Three kinds of error distributions (rows) are shown for the ICSG3D (first column) and LCS6Å (second column) datasets described by medians (points) and 68-percent ranges (colored regions) similar to means $\pm\sigma$ ranges. **a, b,** Distributions of signed errors between detected and actual numbers of atoms. **c, d,** Distributions of position errors according to number of atoms averaged over individual structures. **e, f,** Distributions of species errors according to number of atoms averaged over individual structures. For statistical reliability, we omit the $x$-axis points counting less than 10 materials samples from the visualization (see Section 4.1 for detailed dataset statistics).

# FIG.24

numbers of detected and actual atoms. These results indicate that both methods correctly estimate the number of atoms for most ($i.e.$, > 68 percent) of the samples in the ICSG3D dataset (Fig. 4a). However, the ICSG3D method underestimates the number of atoms for the LCS6Å dataset (Fig. 4b). Likewise, Figs. 4c and 4d show the distributions of position errors and Figs. 4e and 4f show the distributions of species errors. Because the number of atoms is either correctly estimated or underestimated by both methods in most cases, we report the errors in the actual metrics. Examining the distributions at $x = 2$ in Fig. 4e, we can determine the trends of species errors for the diatomic structures in the ICSG3D dataset. The proposed method provides the correct species of both atoms for more than 68 percent of the diatomic materials, whereas ICSG3D often misestimates the species of one of the two atoms.

Overall, although the three types of errors by both methods tend to increase with the number of atoms, our method outperforms ICSG3D consistently for materials with varying numbers of atoms. Compared with our method, the performance of ICSG3D tends to degrade more notably for materials with many atoms. Because ICSG3D often underestimates the number of atoms (Fig. 4b), this overall degradation trend suggests that ICSG3D cannot capture those many-atom structures owing to its spatial resolution limited to $32 \times 32 \times 32$ voxels.

We believe that the notable high performance of the proposed method is attributable to two reasons. First, our method does not use discretization, being advantageous over the grid-based ICSG3D for estimating complex crystal structures. In a grid-based method, the spatial resolution is limited by the cubically increasing computations and memory usage. The proposed NeSF is free from such tradeoff between resolution and computational complexity, and it can thus effectively represent complex structures. Second, the model size of the proposed NeSF using MLPs is much smaller than that of the 3D CNN architecture of ICSG3D. In general, the number of training samples required for an ML model is correlated with the number of trainable parameters. Grid-based methods use layers of 3D convolution filters that involve many trainable parameters. By contrast, the NeSF employs implicit neural representations to indirectly describe the 3D space as a field instead of voxels. Thus, it is efficiently implemented by MLPs with fewer parameters than a 3D CNN. Specifically, the NeSF-based autoencoder has 0.76 million parameters, which is only 2.24% of the number of parameters in the 3D CNN-based ICSG3D (34 million parameters). This difference can make the NeSF advantageous over grid-based methods, especially on small datasets such as the YBCO-like dataset.

## 2.4 Latent space interpolation

We qualitatively analyzed the characteristics of the proposed NeSF-based autoencoder by inspecting the learned latent space of the crystal structures. In general, a good latent space should map similar items (in terms of properties, characteristics, categories, etc.) closely in the space, thus providing latent data representations that facilitate analysis by humans and machines. To assess the construction of the latent space of crystal structures, we visualized transitions in the latent space as sequences of crystal structures. If the latent space is trained to capture relationships between materials in terms of structural similarity, interpolating between two points in the latent space should produce a sequence of materials with similar crystal structures.

Interpolation analysis proceeded as follows:

1. Select two known crystal structures from the ICSG3D test set as the source and destination materials and obtain their latent vectors as $z_{src}$ and $z_{dst}$ via the trained encoder.

2. Interpolate between $z_{src}$ and $z_{dst}$ linearly in the latent space to obtain a sequence of latent vectors.

3. Decode each latent vector via the trained decoder (NeSF) to obtain its crystal structure.

Because the intermediate crystal structures between the source and destination are reconstructed from the latent vectors via the trained decoder, these structures may not appear in the dataset.

# FIG.25

**Figure 5.** Results of interpolation from ZnS to CdS.

**Figure 6.** Results of interpolation from MgO to NaCl.

To facilitate interpretation of the analysis, we chose the well-known zinc-blende and rock-salt structure families as benchmark materials. Both families have compositions given by AX, where A is a cation and X is an anion, and the crystal structure is based on a cubic crystal system. Therefore, if the source and destination materials belong to one of these families, the characteristic composition and structural prototype should be preserved throughout the interpolation path.

As a first example, Fig. 5 shows the results of interpolation from ZnS (mp-10695) to CdS (mp-2469). The obtained transition in the compositional formula is ZnS $\rightarrow$ MgZn$_3$S$_4$ (Mg$_{0.25}$Zn$_{0.75}$S) $\rightarrow$ MgZnS$_2$ (Mg$_{0.5}$Zn$_{0.5}$S) $\rightarrow$ Mg$_3$ZnS$_4$ (Mg$_{0.75}$Zn$_{0.25}$S) $\rightarrow$ MgS $\rightarrow$ MgCd$_3$S$_4$ (Mg$_{0.25}$Cd$_{0.75}$S) $\rightarrow$ CdS.

As a second example, Fig. 6 shows the results of interpolation from MgO (mp-1265) to NaCl (mp-22862). The obtained transition in the compositional formula is MgO $\rightarrow$ NaMgO$_2$ (Na$_{0.5}$Mg$_{0.5}$O) $\rightarrow$ NaO $\rightarrow$ Na$_2$ClO (NaCl$_{0.5}$O$_{0.5}$) $\rightarrow$ Na$_4$Cl$_3$O (NaCl$_{0.75}$O$_{0.25}$) $\rightarrow$ NaCl.

Additionally, we provide the results of interpolation from NaCl (mp-22862) to PbS, from MgO (mp-1265) to CaO (mp-2605), and from PbS (mp-21276) to CaO (mp-2605) in the SI.

In the interpolation examples, composition AX and the cubic structure are mostly preserved. Furthermore, the compositions change continuously without collapsing along the interpolation paths. These results suggest that our encoder learns meaningful continuous representations of crystal structures by capturing their characteristics in an abstract space, and the proposed NeSF model successfully decodes these representations into crystal structures.

# FIG.26

## 2.5 Limitations and future directions

This study was mainly focused on developing a fundamental approach for crystal structure estimation using implicit neural representations. To suggest room for further improvement and important directions in future work, we identified three main limitations of the NeSF.

First, the NeSF adopts a relatively simple network architecture, and architectural design choices were not thoroughly explored. For example, the NeSF treats the elements as mutually independent categorical (one-hot) vectors. Thus, we do not explicitly train our model with the physical characteristics of elements or their similarities. Meanwhile, other developments have attempted to explicitly inject physical characteristics and features of elements into their models. For instance, existing crystal-structure encoders[16] represent input elements using their fingerprints, such as their groups and periods, electron densities, atomic radii, and electronegativities, instead of using one-hot vectors. In ICSG3D[24], the outputs of atomic species are trained using the mean squared error of the atomic numbers rather than a categorical loss, which is considered in our method. Incorporating these schemes into our model may allow to reflect the characteristics and similarities between elements in the latent space and reconstructed crystal structures. Another architectural choice regarding the NeSF is whether to use the conventional (current choice) or primitive unit cell for expressing crystal structures. Both formats have their advantages and drawbacks depending on the application. The conventional unit cell offers more intuitive visualizations that may facilitate manual analysis, whereas the primitive unit cell provides more compact structure representations that may facilitate computer processing. A thorough analysis of the network architecture design should be performed in future work.

Another limitation is that the proposed NeSF does not explicitly consider space-group symmetry. Thus, the local spatial arrangements of atoms in conventional unit cells estimated by the NeSF do not necessarily obey space-group symmetry. Although ICSG3D[24] shares the same limitation, symmetry is an important concept in crystallography. Thus, incorporating the constraint of space-group symmetry into the NeSF is an important direction of future work.

Finally, for an evaluation purpose, we adopted the autoencoder architecture rather than generative models, such as variational autoencoders[16,25,37] and generative adversarial networks[26,37–39]. These generative models intentionally perturb latent structural representations to produce diverse structures that do not appear in the dataset. While this aspect of the generative models is more suitable for novel structure discovery, the lack of ground-truth structures prevents quantitative and reliable performance analysis. The NeSF should be applied to generative models in future work with appropriate performance analysis.

## 3 Conclusion

We propose the NeSF to estimate crystal structures using neural networks. Determining crystal structures directly using neural networks is challenging because these structures are essentially represented as an unordered set with varying numbers of atoms. NeSF overcomes this difficulty by treating the crystal structure as a continuous vector field rather than as a discrete set of atoms. We borrow the idea of the NeSF from vector fields in physics and the recent implicit neural representations in computer vision. An implicit neural representations is an ML technique to represent 3D geometries using neural networks. The NeSF extends this technique by introducing the position and species fields to estimate the atomic positions and species of crystal structures, respectively. Unlike existing grid-based approaches for representing crystal structures, the NeSF is free from the tradeoff between spatial resolution and computational complexity and can represent any crystal structure.

The NeSF was applied as an autoencoder for crystal structures and demonstrated its performance and expressive power on datasets with diverse crystal structures. A quantitative performance analysis showed a clear advantage of the NeSF-based autoencoder over an existing grid-based method, especially for estimating complex crystal structures. Furthermore, a qualitative analysis of the learned latent space revealed that the autoencoder captures similarities between crystal structures rather than mapping crystal structures randomly.

# FIG.27

In materials science, the design and construction of crystal structures are fundamental processes when searching for materials with the desired properties. ML is advancing rapidly with the development of neural networks, and representing arbitrary crystal structures using those networks is essential for next-generation development of materials. For instance, the NeSF can be easily incorporated into powerful deep generative models, such as variational autoencoders and generative adversarial networks, to discover novel crystal structures. Such generative models for crystal structures will be important for inverse design of materials, which is a major challenge in MI. We believe that the NeSF can overcome the technical bottleneck of ML for crystal structure estimation and pave the way for next-generation development of materials.

## 4 Methods

### 4.1 Datasets

We evaluated the proposed NeSF-based autoencoder using datasets from the Materials Project.

- ICSG3D dataset: To compare with the ICSG3D baseline, we reproduced the dataset used in the original paper[24]. Unfortunately, the proponents of ICSG3D did not specify the identifiers of the materials used for their experiments. Therefore, by following the procedures described in their paper, we crawled the three material classes with cubic structures, namely, binary alloys (AB), ternary perovskites (ABX$_3$), and Heusler compounds (ABX$_2$), from the Materials Project. Then, we split the dataset into training (7194 samples), validation (342 samples), and test (360 samples) sets. The numbers of atoms per unit cell are listed in Table 2.

- LCS6Å dataset: From the Materials Project, we collected all the materials samples with unit cell sizes of 6 Å or less along the $x$, $y$, and $z$ axes. Unlike the ICSG3D dataset, the LCS6Å dataset has no restriction on material classes and prototypes and covers 4.31% of the Materials Project database. This dataset was also split into training (5418 samples), validation (286 samples), and test (301 samples) sets, and the numbers of atoms in the samples are listed in Table 3.

- YBCO-like dataset: From the Materials Project, we extracted 100 samples with structures similar to YBCO (YBa$_2$Cu$_3$O$_7$), that is, narrow crystal structures along the $c$ axis. The structures of these materials have narrow and anisotropic unit cells and contain various elements. Given the complexity of the structures and limited number of samples, this dataset is considered the hardest and most practical among the three evaluated datasets. This dataset was also split into training (90 samples), validation (5 samples), and test (5 samples) sets. The numbers of atoms of the samples are listed in Table 4.

All the crystal structures were described by a conventional cell for the input and output of the autoencoders.

### Table 2. Numbers of atoms per unit cell in ICSG3D dataset

| No. atoms per cell | 2 | 5 | 6 | 8 | 12 | 15 | 16 | 20 | 32 | 40 |
|---|---|---|---|---|---|---|---|---|---|---|
| Training | 184 | 1413 | 7 | 572 | 1 | 12 | 4616 | 17 | 18 | 54 |
| Validation | 18 | 66 | | 32 | | | 223 | 1 | | 2 |
| Test | 24 | 61 | | 34 | | | 237 | | | 4 |

### Table 3. Numbers of atoms per unit cell in LCS6Å dataset

| No. atoms per cell | 1 | 2 | 3 | 4 | 5 | 6 | 7 | 8 | 9 | 10 | 11 | 12 | 13 | 14 | 15 | 16 | 18 | 20 | 21 | 22 | 24 | 28 |
|---|---|---|---|---|---|---|---|---|---|---|---|---|---|---|---|---|---|---|---|---|---|---|
| Training | 21 | 599 | 44 | 1411 | 1325 | 244 | 69 | 686 | 58 | 137 | 9 | 352 | 9 | 24 | 4 | 402 | 5 | 6 | 1 | 2 | 9 | 1 |
| Validation | | 35 | 2 | 80 | 81 | 14 | 3 | 41 | 4 | 5 | | 13 | | 2 | | 20 | | | | | 1 | |
| Test | | 30 | 3 | 83 | 68 | 17 | 2 | 35 | | 5 | | 19 | | 1 | | 23 | | | | | | |

# FIG.28

### Table 4. Numbers of atoms per unit cell in YBCO-like dataset

| No. atoms per cell | 13 |
|---|---|
| Training | 90 |
| Validation | 5 |
| Test | 5 |

## 4.2 Neural network architecture

The proposed NeSF-based autoencoder consists of simple MLPs. Specifically, the encoder first transforms the atomic position and species of each atom into a 512-dimensional feature vector. These feature vectors per atom are then aggregated into a single feature vector via a max pooling and converted into a 192-dimensional feature vector, which represents latent vector $z$ of the input crystal structure. Given $z$ as the input, the decoder estimates four types of structural information features using four separate MLPs. The atomic positions are estimated via the position field implemented as an MLP with nine fully connected layers. The atomic species are estimated via the ppecies field implemented as an MLP with three fully connected layers. Lattice lengths $(a,b,c)$ and angles $(\alpha,\beta,\gamma)$ are estimated using the corresponding MLPs with two fully connected layers. More details on the architecture are provided in the SI.

## 4.3 Training

During training, the four types of outputs from the decoder (*i.e.*, atomic position, species, lattice lengths, and angles) were evaluated using the following loss function:

$$L = \lambda_{pos}L_{pos} + \lambda_{spe}L_{spe} + \lambda_{len}L_{len} + \lambda_{ang}L_{ang} \tag{2}$$

where $L_{pos}$ is the mean squared error between the estimated and true atomic positions, $L_{spe}$ is the cross-entropy loss function for the atomic species distributions, and $L_{len}$ and $L_{ang}$ are the mean squared error of the lattice lengths and angles, respectively. The total loss function, $L$, is given by the weighted sum of these loss functions with weights $(\lambda_{pos},\lambda_{spe},\lambda_{len},\lambda_{ang}) = (10,0.1,1,1)$. We optimized the loss function using stochastic gradient descent with a batch size of 128. We used Adam as the optimizer with an initial learning rate of $10^{-3}$ decaying every 640 epochs by a factor of 0.5. For each dataset, we conducted iterative training for 3200 epochs for all the materials samples in the dataset. We applied early stopping based on the validation score for both the proposed method and ICSG3D, except for the YBCO-like dataset, for which the few validation samples may lead to unreliable validation scores. The training of the NeSF model on the LCS6Å dataset took approximately 9 h using a computer equipped with a single NVIDIA RTX8000 graphics processor. For details regarding our strategies for validating the trained models and tuning the hyperparameters (*e.g.*, latent dimensionality, number of network layers, training batch size), see the SI.

## References

1. De Graef, M. & McHenry, M. E. *Structure of Materials*. An Introduction to Crystallography, Diffraction and Symmetry (Cambridge University Press, 2012).

2. Callister, W. D. & Rethwisch, D. G. *Materials Science and Engeneering* (John Wiley and Sons, 2010).

3. Lookman, T., Alexander, F. J. & Rajan, K. *Information Science for Materials Discovery and Design* (Springer, 2015).

4. Schmidt, J., Marques, M. R. G., Botti, S. & Marques, M. A. L. Recent advances and applications of machine learning in solid-state materials science. *npj Comput. Mater* 5, 1–36, DOI: 10.1038/s41524 -019-0221-0 (2019).

# FIG.29

5. Szymanski, N. J. *et al.* Toward autonomous design and synthesis of novel inorganic materials. *Mater. Horiz.* **8**, 2169–2198, DOI: 10.1039/D1MH00495F (2021).

6. Choudhary, K. *et al.* Recent advances and applications of deep learning methods in materials science. *npj Comput. Mater* **8**, 1–26, DOI: 10.1038/s41524-022-00734-6 (2022).

7. Agrawal, A. & Choudhary, A. Perspective: Materials informatics and big data: Realization of the "fourth paradigm" of science in materials science. *APL Mater.* **4**, 053208, DOI: 10.1063/1.4946894 (2016).

8. Jha, D. *et al.* ElemNet: Deep Learning the Chemistry of Materials From Only Elemental Composition. *Sci. Reports* **8**, 17593, DOI: 10.1038/s41598-018-35934-y (2018).

9. Goodall, R. E. A. & Lee, A. A. Predicting materials properties without crystal structure: Deep representation learning from stoichiometry. *Nat Commun* **11**, 6280, DOI: 10.1038/s41467-020-19964-7 (2020).

10. Park, C. W. & Wolverton, C. Developing an improved crystal graph convolutional neural network framework for accelerated materials discovery. *Phys. Rev. Mater.* **4**, 063801, DOI: 10.1103/PhysRe vMaterials.4.063801 (6    1, 2020).

11. Wang, A. Y.-T., Kauwe, S. K., Murdock, R. J. & Sparks, T. D. Compositionally restricted attention-based network for materials property predictions. *npj Comput. Mater* **7**, 1–10, DOI: 10.1038/s41524 -021-00545-1 (2021).

12. Park, W. B. *et al.* Classification of crystal structure using a convolutional neural network. *IUCrJ* **4**, 486–494, DOI: 10.1107/S205225251700714X (2017).

13. Oviedo, F. *et al.* Fast and interpretable classification of small X-ray diffraction datasets using data augmentation and deep neural networks. *npj Comput. Mater.* **5**, 60 (2019).

14. Szymanski, N. J., Bartel, C. J., Zeng, Y., Tu, Q. & Ceder, G. Probabilistic Deep Learning Approach to Automate the Interpretation of Multi-phase Diffraction Spectra. *Chem. Mater.* **33**, 4204–4215, DOI: 10.1021/acs.chemmater.1c01071 (2021).

15. Tshitoyan, V. *et al.* Unsupervised word embeddings capture latent knowledge from materials science literature. *Nature* **571**, 95–98, DOI: 10.1038/s41586-019-1335-8 (2019).

16. Xie, T. & Grossman, J. C. Crystal Graph Convolutional Neural Networks for an Accurate and Interpretable Prediction of Material Properties. *Phys. Rev. Lett.* **120**, 145301, DOI: 10.1103/Phys RevLett.120.145301 (2018).

17. Cheng, J., Zhang, C. & Dong, L. A geometric-information-enhanced crystal graph network for predicting properties of materials. *Commun Mater* **2**, 1–11, DOI: 10.1038/s43246-021-00194-3 (2021).

18. Choudhary, K. & DeCost, B. Atomistic Line Graph Neural Network for improved materials property predictions. *npj Comput. Mater* **7**, 1–8, DOI: 10.1038/s41524-021-00650-1 (2021).

19. Noh, J. *et al.* Inverse Design of Solid-State Materials via a Continuous Representation. *Matter* **1**, 1370–1384, DOI: 10.1016/j.matt.2019.08.017 (2019).

20. Noh, J., Gu, G. H., Kim, S. & Jung, Y. Machine-enabled inverse design of inorganic solid materials: Promises and challenges. *Chem. Sci.* **11**, 4871–4881, DOI: 10.1039/D0SC00594K (2020).

21. Yao, Z. *et al.* Inverse design of nanoporous crystalline reticular materials with deep generative models. *Nat. Mach. Intell.* **3**, 76–86, DOI: 10.1038/s42256-020-00271-1 (2021).

22. Long, T. *et al.* Constrained crystals deep convolutional generative adversarial network for the inverse design of crystal structures. *npj Comput. Mater* **7**, 1–7, DOI: 10.1038/s41524-021-00526-4 (2021).

# FIG.30

23. Fung, V., Zhang, J., Hu, G., Ganesh, P. & Sumpter, B. G. Inverse design of two-dimensional materials with invertible neural networks. *npj Comput. Mater* **7**, 1–9, DOI: 10.1038/s41524-021-00670-x (2021).

24. Court, C. J., Yildirim, B., Jain, A. & Cole, J. M. 3-D Inorganic Crystal Structure Generation and Property Prediction via Representation Learning. *J. Chem. Inf. Model.* **60**, 4518–4535, DOI: 10.1021/acs.jcim.0c00464 (2020).

25. Hoffmann, J. *et al.* Data-driven approach to encoding and decoding 3-d crystal structures. *arXiv preprint arXiv:1909.00949* (2019).

26. Kim, S., Noh, J., Gu, G. H., Aspuru-Guzik, A. & Jung, Y. Generative Adversarial Networks for Crystal Structure Prediction. *ACS Cent. Sci.* **6**, 1412–1420, DOI: 10.1021/acscentsci.0c00426 (2020).

27. Chen, C., Ye, W., Zuo, Y., Zheng, C. & Ong, S. P. Graph Networks as a Universal Machine Learning Framework for Molecules and Crystals. *Chem. Mater.* **31**, 3564–3572, DOI: 10.1021/acs.chemmater.9b01294 (2019).

28. Charles, R. Q., Su, H., Kaichun, M. & Guibas, L. J. Pointnet: Deep learning on point sets for 3d classification and segmentation. In *IEEE/CVF Conference on Computer Vision and Pattern Recognition (CVPR)* (2017).

29. Zaheer, M. *et al.* Deep sets. In *Neural Information Processing Systems* (2017).

30. Mildenhall, B. *et al.* NeRF: Representing scenes as neural radiance fields for view synthesis. *arXiv* (2020). 2003.08934.

31. Park, J. J., Florence, P., Straub, J., Newcombe, R. & Lovegrove, S. Deepsdf: Learning continuous signed distance functions for shape representation. In *IEEE/CVF Conference on Computer Vision and Pattern Recognition (CVPR)* (2019).

32. Chen, Z. & Zhang, H. Learning implicit fields for generative shape modeling. In *Proceedings of the IEEE/CVF Conference on Computer Vision and Pattern Recognition (CVPR)* (2019).

33. Mescheder, L., Oechsle, M., Niemeyer, M., Nowozin, S. & Geiger, A. Occupancy networks: Learning 3d reconstruction in function space. In *Proceedings of the IEEE/CVF Conference on Computer Vision and Pattern Recognition (CVPR)* (2019).

34. Xie, Y. *et al.* Neural fields in visual computing and beyond. *Comput. Graph. Forum* DOI: 10.1111/cgf.14505 (2022).

35. Duan, Y. *et al.* Curriculum deepsdf. In Vedaldi, A., Bischof, H., Brox, T. & Frahm, J.-M. (eds.) *Computer Vision – ECCV 2020*, 51–67 (Springer International Publishing, Cham, 2020).

36. Kingma, D. P. & Ba, J. Adam: A method for stochastic optimization. In *The International Conference on Learning Representations (ICLR)* (2015).

37. Goodfellow, I. J., Bengio, Y. & Courville, A. *Deep Learning* (MIT Press, Cambridge, MA, USA, 2016). http://www.deeplearningbook.org.

38. Goodfellow, I. *et al.* Generative adversarial nets. In Ghahramani, Z., Welling, M., Cortes, C., Lawrence, N. & Weinberger, K. (eds.) *Advances in Neural Information Processing Systems*, vol. 27 (Curran Associates, Inc., 2014).

39. Nouira, A., Sokolovska, N. & Crivello, J.-C. CrystalGAN: Learning to Discover Crystallographic Structures with Generative Adversarial Networks. *arXiv:1810.11203 [cs, stat]* (2019). 1810.11203.

## Data availability

The codes, documents, and supplementary resources that support the findings of this study are available at https://hogehoge.

# EP 4 625 255 A1

## INTERNATIONAL SEARCH REPORT

| | International application No. |
|---|---|
| | **PCT/JP2023/040970** |

### A. CLASSIFICATION OF SUBJECT MATTER

*G06N 3/0455*(2023.01)i; *G16C 20/70*(2019.01)i; *G16C 20/80*(2019.01)i
FI:   G06N3/0455; G16C20/70; G16C20/80

According to International Patent Classification (IPC) or to both national classification and IPC

### B. FIELDS SEARCHED

Minimum documentation searched (classification system followed by classification symbols)

G06N3/0455; G16C20/70; G16C20/80

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2021/262792 A1 (SRI INTERNATIONAL) 30 December 2021 (2021-12-30)<br>  paragraphs [0083], [0085], [0091], [0100] | 1, 7, 9-12 |
| Y | | 2-4 |
| A | | 5-6, 8 |
| P, X | CHIBA, Naoya et al. Neural Structure Fields with Application to Crystal Structure Autoencoders. arXiv.org [online]. Cornell University. 08 December 2022, [retrieved on 18 January 2024], pp. 1-29, internet:<URL: https://arxiv.org/pdf/2012.13120v1.pdf><br>  entire text, all drawings | 1, 7, 9-12 |
| P, Y | | 2-4 |
| P, A | | 5-6, 8 |
| A | JP 2020-166706 A (X ABILITY CO LTD) 08 October 2020 (2020-10-08)<br>  paragraphs [0058]-[0060] | 1-12 |
| A | JP 2018-010428 A (HITACHI LTD) 18 January 2018 (2018-01-18)<br>  paragraph [0025] | 1-12 |

✓ Further documents are listed in the continuation of Box C.    ✓ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **18 January 2024** | **30 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2023/040970** |

**C.    DOCUMENTS CONSIDERED TO BE RELEVANT**

| Category* | Citation of document, with indication, where appropriate, of the relevant passages | Relevant to claim No. |
| --- | --- | --- |
| P, A | WO 2023/047843 A1 (OMRON CORPORATION) 30 March 2023 (2023-03-30) abstract | 1-12 |
| P, A | CN 116343937 A (SHENZHEN XTAIPI TECHNOLOGY CO., LTD.) 27 June 2023 (2023-06-27) abstract | 1-12 |

Form PCT/ISA/210 (second sheet) (July 2022)

## INTERNATIONAL SEARCH REPORT
### Information on patent family members

International application No.

**PCT/JP2023/040970**

| Patent document cited in search report | | | Publication date (day/month/year) | Patent family member(s) | | | Publication date (day/month/year) |
|---|---|---|---|---|---|---|---|
| WO | 2021/262792 | A1 | 30 December 2021 | JP 2023-541737 A <br> paragraphs [0083], [0085], [0091], [0100] <br> US 2023/0103487 A1 | | | |
| JP | 2020-166706 | A | 08 October 2020 | WO 2020/203922 A1 <br> paragraphs [0058]-[0060] | | | |
| JP | 2018-010428 | A | 18 January 2018 | US 2018/0018408 A1 <br> paragraph [0044] | | | |
| WO | 2023/047843 | A1 | 30 March 2023 | JP 2023-47983 A <br> abstract | | | |
| CN | 116343937 | A | 27 June 2023 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022186043 A

- JP 2023130520 A

**Non-patent literature cited in the description**

- **CALLUM J. COURT ; BATUHAN YILDIRIM ; APOORV JAIN ; JACQUELINE M. COLE**. 3-D Inorganic Crystal Structure Generation and Property Prediction via Representation Learning. *J. Chem. inf. Model.*, 2020, vol. 60 (10), 4518-4535, https://pubs.acs.org/doi/10.1021/acs.jcim.0c00464